# EUROPEAN PATENT APPLICATION

(11) **EP 1 676 837 A1**
(43) Date of publication of application: **05.07.2006**
(21) Application number: 04792414.7
(22) Date of filing: 07.10.2004
(51) Int. Cl.: C07D 207/10, C07C 237/44, C07C 271/28, C07C 269/06, C07C 269/04

(54) **PROCESS FOR PRODUCING AMINOPYRROLIDINE DERIVATIVE AND INTERMEDIATE COMPOUND**

(30) Priority: 08.10.2003 JP 2003349318; 09.10.2003 JP 2003350441; 09.10.2003 JP 2003350439
(71) Applicant: Teijin Pharma Limited, Tokyo 100-0011 (JP)
(72) Inventor: TAKEYASU, Takumi, c/o Teijin Pharma Limited, Iwakuni-shi, Yamaguchi 7400014 (JP); SATO, Yoshinori, c/o Teijin Pharma Limited, Iwakuni-shi, Yamaguchi 7400014 (JP); IMAI, Minoru, c/o Teijin Pharma Limited, Tokyo 1000011 (JP); SAKAI, Mitsuru, c/o Teijin Pharma Limited, Hino-shi, Tokyo 1910065 (JP); MANABE, Kenji, Hino-shi, Tokyo 1910065 (JP); MATSUMOTO, Yoshiyuki, c/o Teijin Pharma Limited, Hino-shi, Tokyo 1910065 (JP); TAKEUCHI, Susumu, c/o Teijin Pharma Limited, Hino-shi Tokyo 1910065 (JP); KAWANA, Asahi, c/o Teijin Pharma Limited, Iwakuni-shi, Yamaguchi 7400014 (JP); KOGA, Masahiro, c/o Teijin Pharma Limited, Hino-shi, Tokyo 1910065 (JP); ASAHIDA, Mitsuharu, Kai-shi, Yamanashi 4000124 (JP)
(74) Representative: Hallybone, Huw George
(86) International application number: PCT/JP2004/015186
(87) International publication number: WO 2005/035493

(57) **Abstract**

There is provided an industrial production method of an aminopyrrolidine derivative having chemokine receptor antagonist activity represented by the following formula, a synthetic intermediate thereof and a production method thereof: 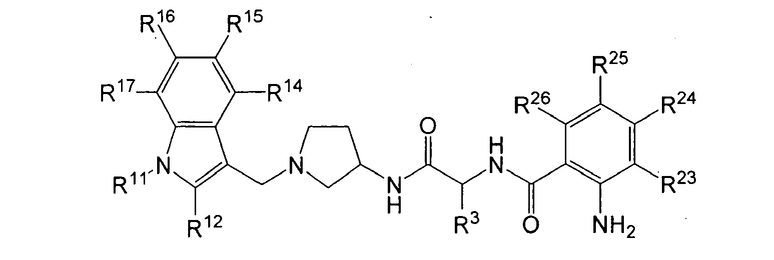 wherein R¹¹ is H, C₁-C₆ alkyl or C₂-C₇ alkanoyl; R¹², R¹⁴, R¹⁵, R¹⁶ and R¹⁷ are H, halogen, optionally halogenated C₁-C₆ alkyl, optionally halogenated C₁-C₆ alkoxy, hydroxyl or C₂-C₇ alkoxycarbonyl; R²³, R²⁴, R²⁵ and R²⁶ are H, halogen, optionally halogenated C₁-C₆ alkyl, optionally halogenated C₁-C₆ alkoxy or hydroxyl; and R³ is H or C₁-C₆ alkyl.

## Description

### FIELD OF THE INVENTION

The present invention relates to a method for producing aminopyrrolidine derivatives. More specifically, it relates to a method for producing chemokine receptor antagonists that are expected as effective for treating and/or preventing diseases in which infiltration of leukocyte components such as monocyte and lymphocyte into tissues plays a major role in their progress and preservation.

Furthermore, the present invention relates to anthranilamides that are useful intermediates in the synthesis of the aminopyrrolidine derivatives and a method for production thereof.

### BACKGROUND ART

Chemokines such as MIP-1α and MCP-1 are protein factors that cause migration and activation of leukocytes. It is known that their function is expressed via mediation by chemokine receptors on leukocytes (Allergy & Immunology, 1999, vol. 6, no. 11). It is, therefore, expected that a chemokine receptor antagonist, which can inhibit activity of chemokines onto target cells, is effective for treating and/or preventing one or more diseases in which infiltration of leukocytes into tissues plays a major role in their progress and preservation, such as arteriosclerosis, rheumatoid arthritis, psoriasis, asthma, ulcerous colitis, nephritis (nephropathy), multiple sclerosis, pulmonary fibrosis, cardiomyopathy, hepatitis, pancreatitis, sarcoidosis, Crohn's disease, endometriosis, congestive heart failure, viral meningitis, cerebral infarction, neuropathy, Kawasaki disease, sepsis, allergic rhinitis and allergic dermatitis (Schwartz, M.K. et al., Exp. Opin. Ther. Patents, 1999, 9, 1471). Based on this findings investigation towards development of chemokine receptor antagonists has progressed, and a cyclic amine derivative having high activity as a chemokine receptor antagonist was found (WO 99/25686).

As methods for producing aminopyrrolidine derivatives having such chemokine receptor antagonist activity, examples of syntheses for compounds having similar structure are disclosed in WO 99/25686 and WO 98/50534. However, the methods reported therein are not simple, and the disclosed examples are not suitable for large-scale synthesis for reasons such as use of dichloromethane as solvent.

Furthermore, because many of preferred compounds having chemokine receptor antagonist activity contain anthranilamide skeletal structure, in order to obtain a wide variety of compounds having chemokine receptor antagonist activity, it was desired to produce synthetic intermediates efficiently by a method suitable for large-scale synthesis.

As an example of compound having such anthranilamide-like structure, a 2-phenylaminobenzamide derivative is disclosed in WO 01/05392 and WO 00/37141. However, the characteristic feature of this compound is its hydroxamic acid structure, and the above-mentioned compound having chemokine receptor antagonist activity cannot be derived from this compound.

Further, although an amide compound that inhibits chemokine receptor is disclosed in WO 02/60859 and WO 02/50019, such a compound with anthranilic acid structure is not specifically disclosed, and therefore a different synthetic method is required.

### DISCLOSURE OF THE INVENTION

An object of the present invention is to provide a simple and industrial production method for the compounds that are chemokine receptor antagonists disclosed in WO 99/25686. In other words, it is to provide a method by which the desired aminopyrrolidine derivatives can be produced in high yields under mild conditions by avoiding the use of halogenated solvent which imposes environmental load.

Furthermore, another object of the present invention is to find intermediates suitable for producing the compounds disclosed in WO 99/25686, particularly, intermediates suitable for producing the anthranilamide skeletal moiety. Still another object is to provide a method suitable for industrial production thereof.

The present investors have investigated with the above objects, found production methods of the present invention, intermediates and methods for production thereof, and accomplished the present invention.

Namely, the first invention is a method for producing aminopyrrolidine derivatives or salts thereof comprising reaction steps 1 and 2 represented by the following reaction formula (I):

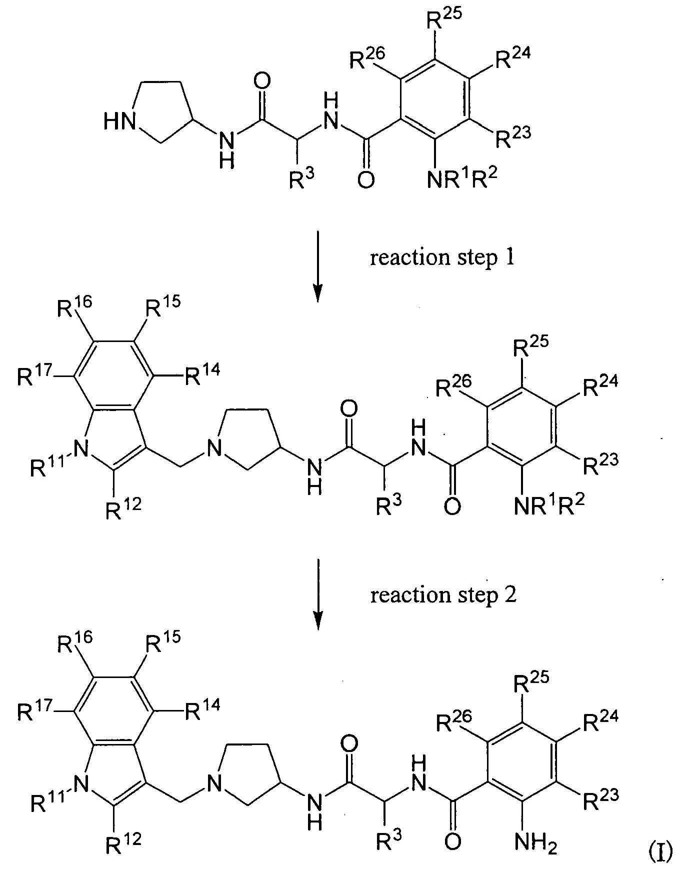

In formula (I), R¹ and R² represent independently hydrogen or a protecting group for amino group (wherein R¹ and R² may, taken together, form a cyclic structure);
R³ represents hydrogen or C₁-C₆ alkyl;
R¹¹ represents hydrogen, C₁-C₆ alkyl or C₂-C₇ alkanoyl (carbonyl carbon is included in the number of carbon atoms);
R¹², R¹⁴ , R¹⁵, R¹⁶ and R¹⁷ represent independently hydrogen, halogen, optionally halogenated
C₁-C₆ alkyl, optionally halogenated C₁-C₆ alkoxy, hydroxyl or C₂-C₇ alkoxycarbonyl (carbonyl carbon is included in the number of carbon atoms); and
R²³, R²⁴, R²⁵ and R²⁶ represent independently hydrogen, halogen, optionally halogenated C₁-C₆ alkyl, optionally halogenated C₁-C₆ alkoxy or hydroxyl.

In the case where both R¹ and R² are hydrogen, since reaction step 2 is not necessary, the present invention is a method for producing aminopyrrolidine derivatives or salts thereof comprising only reaction step 1 in the above formula.

Because the aminopyrrolidine derivative thus obtained has a chiral carbon on the pyrrolidine ring, enantiomers may exist. The present invention includes methods for producing any of R-form, S-form and mixtures thereof. When R³ represents C₁-C₆ alkyl, the present invention also includes methods for producing any of the stereoisomers on the carbon atom bonding to R³ or mixtures thereof.

The second invention is a method for producing aminopyrrolidine derivatives or salts thereof comprising a condensation step represented by the following reaction formula (II): 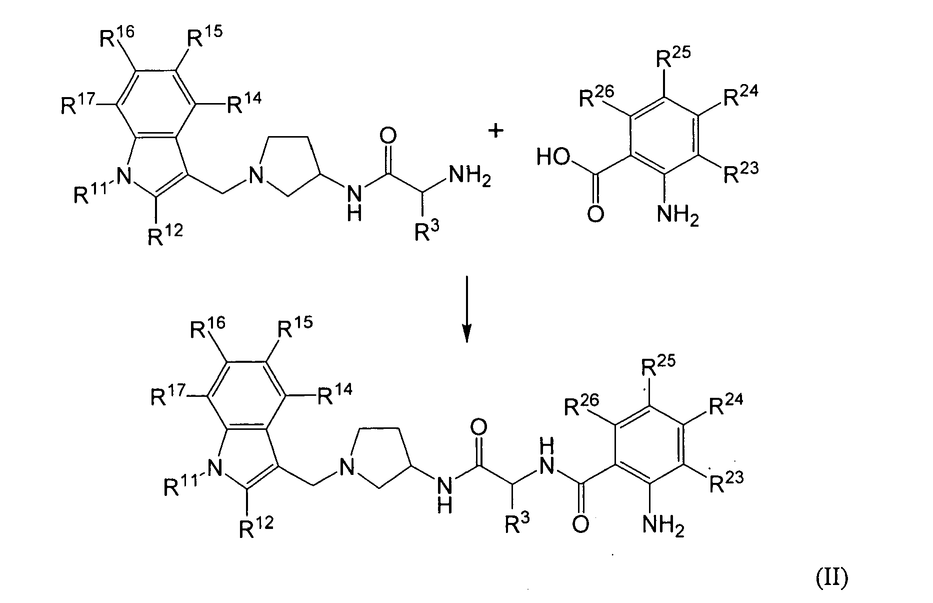 wherein R³, R¹¹, R¹², R¹⁴, R¹⁵ , R¹⁶ R¹⁷, R²³, R²⁴, R²⁵ and R²⁶ in formula (II) are as defined in reaction formula (I).

Because the aminopyrrolidine derivative thus obtained has a chiral carbon on the pyrrolidine ring, enantiomers may exist. The present invention includes methods for producing any of R-form, S-form and mixtures thereof. When R³ represents C₁-C₆ alkyl, the present invention also includes methods for producing any of the stereoisomers on the carbon atom bonding to R³ or mixtures thereof.

The third invention is anthranilamide derivatives or salts thereof which are intermediates for producing the above-mentioned aminopyrrolidine derivatives, represented by the following formula (III): 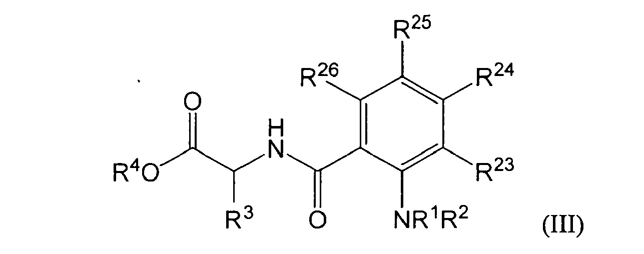 wherein R¹, R², R³, R²³, R²⁴, R²⁵ and R²⁶ in formula (III) are as defined in reaction formula (I), and R⁴ represents hydrogen or C₁-C₆ alkyl.

Because this compound may contain a carboxyl group or a basic nitrogen, it may form various salts, which are also included in the present invention.

Further, when R³ represents C₁-C₆ alkyl, the stereoisomers on the carbon atom bonding to R³ exist. The present invention includes methods for producing any of R-form, S-form and mixtures thereof.

The fourth invention is a method for producing the above-mentioned anthranilamide derivatives or salts thereof comprising a reaction step represented by the following reaction formula (IV): 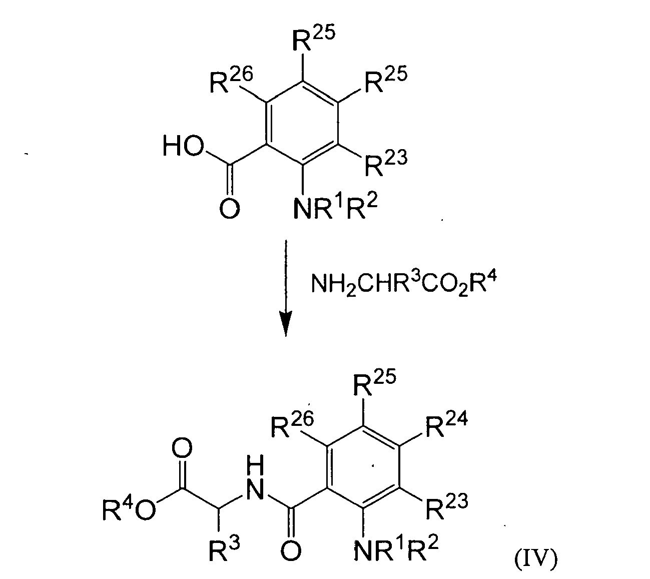 wherein R¹, R², R³, R²³, R²⁴, R²⁵ and R²⁶ in formula (IV) are as defined in formula (I), and R⁴ is the same as defined in formula (III).

Further, when R³ represents C₁-C₆ alkyl, the stereoisomers on the carbon atom bonding to R³ exist. The present invention includes methods for producing any of R-form, S-form and mixtures thereof.

### BEST MODE FOR CARRYING OUT THE INVENTION

In the present specification, "Boc", "Z", "WSC" and "HOBt" represent *t*-butoxycarbonyl, benzyloxycarbonyl, 1-ethyl-3-(3-dimethylaminopropyl)carbodiimide and 1-hydroxy-1,2,3-benzotriazole, respectively.

In the present specification, "halogen" means F, Cl, Br or I.

In each formula in the present specification, R¹ and R² represent independently hydrogen or a protecting group for amino group (wherein R¹ and R² may, taken together, form a cyclic structure). The protecting group for amino group is preferably methoxycarbonyl, *t*-butoxycarbonyl, benzyloxycarbonyl, allyloxycarbonyl, formyl, acetyl, benzoyl, methyl, ethyl, allyl, benzenesulfonyl or phthaloyl. When the protecting group contains an aromatic ring, the aromatic ring may be substituted with one or more of nitro, amino, C₁-C₆ alkyl, C₁-C₆ alkoxy or halogen. In particular, R¹ and R² are preferably hydrogen, *t*-butoxycarbonyl or benzyloxycarbonyl.

In each formula in the present specification, R³ represents hydrogen or C₁-C₆ alkyl. R³ is preferably hydrogen.

In each formula in the present specification, R⁴ represents hydrogen or C₁-C₆ alkyl. R⁴ is preferably hydrogen or methyl, especially preferably hydrogen.

In each formula in the present specification, R¹¹ represents hydrogen, C₁-C₆ alkyl or C₂-C₇ alkanoyl. Preferably R¹¹ is hydrogen.

In each formula in the present specification, R¹², R¹⁴, R¹⁵, R¹⁶ and R¹⁷ represent independently hydrogen, halogen, optionally halogenated C₁-C₆ alkyl, optionally halogenated C₁-C₆ alkoxy, hydroxyl or C₂-C₇ alkoxycarbonyl. Preferably R¹², R¹⁴, R¹⁵ and R¹⁷ are all hydrogen. R¹⁶ is preferably C₁-C₆ alkyl, especially preferably methyl.

In each formula in the present specification, R²³, R²⁴, R²⁵ and R²⁶ represent independently hydrogen, halogen, optionally halogenated C₁-C₆ alkyl, optionally halogenated C₁-C₆ alkoxy or hydroxyl. Preferably R²³, R²⁴ and R²⁶ are all hydrogen. R²⁵ is preferably optionally halogenated C₁-C₆ alkoxy, especially preferably trifluoromethoxy.

Here the first invention is explained. Reaction step 1 in the production method represented by reaction formula (I) is a step by which an indole derivative is introduced into an aminopyrrolidine skeleton. Examples of this step are largely classified into two methods.

One of them is reaction of an indole derivative having no substituent at the 3-position of its indole ring with an amine in the presence of a synthon of formaldehyde such as formalin, paraformaldehyde and trioxane as shown in the following reaction formula.

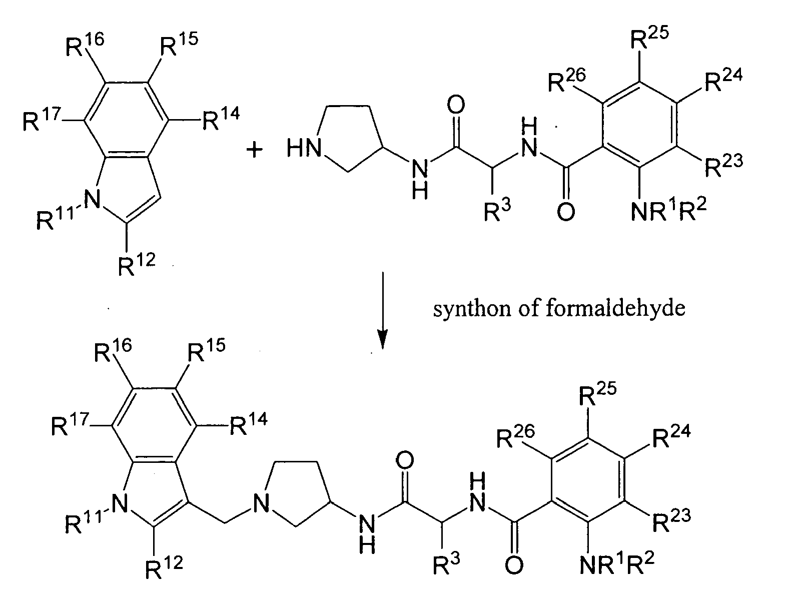

Namely, when an indole derivative having no substituent at the 3-position is treated with an amine in solvent such as acetic acid/1,4-dioxane mixed solvent (mixing ratio is for example 1:1), ethanol, methanol and acetic acid/methanol mixed solvent (with any mixing ratio), condensation reaction proceeds, where the 3-position in the indole derivative and the primary or secondary amine are bonded via one methylene group. In this case, preferred solvents are ethanol, acetic acid and tetrahydrofuran. The reaction temperature is preferably room temperature. As a synthon of formaldehyde, 37% formalin is particularly preferred.

Alternatively, an indole derivative having a dialkylaminomethyl group at the 3-position of the indole ring may be used as shown in the following reaction formula:

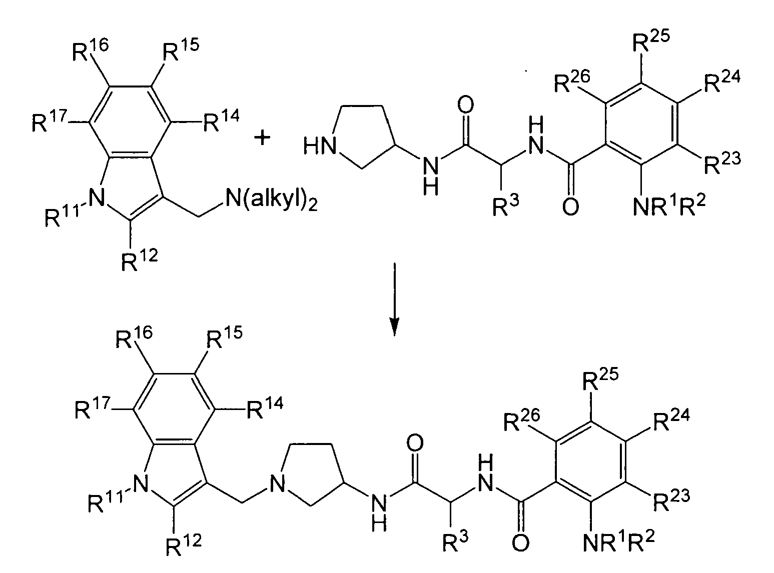

Namely, reaction between the indole derivative and the amine in organic solvent gives the desired indole derivative. As reaction solvent can be used a variety of solvents such as alcohols ethers, esters and hydrocarbons except for ketones or amines. Particularly 2-propanol, propyl acetate and toluene are preferred.

Reaction step 2 in the production method expressed by the reaction formula (I) is deprotection step of the amino group in the anthranilic acid moiety, which is necessary only when protecting group(s) are used.

For example, when *t*-butoxycarbonyl is used as a protecting group, it can be removed in organic solvent by addition of hydrochloric acid, hydrogen chloride solution such as hydrogen chloride/diethyl ether, hydrogen chloride/1,4-dioxane and hydrogen chloride/methanol, or trifluoroacetic acid. In this case, hydrogen chloride/1 ,4-dixane or hydrogen chloride/methanol solution is particularly preferred.

Raw materials to obtain the aminopyrrolidine derivatives of the present invention can be produced, for example, by the following synthetic route, wherein R¹, R², R³, R²³, R²⁴, R²⁵ and R²⁶ in the formula are as defined above.

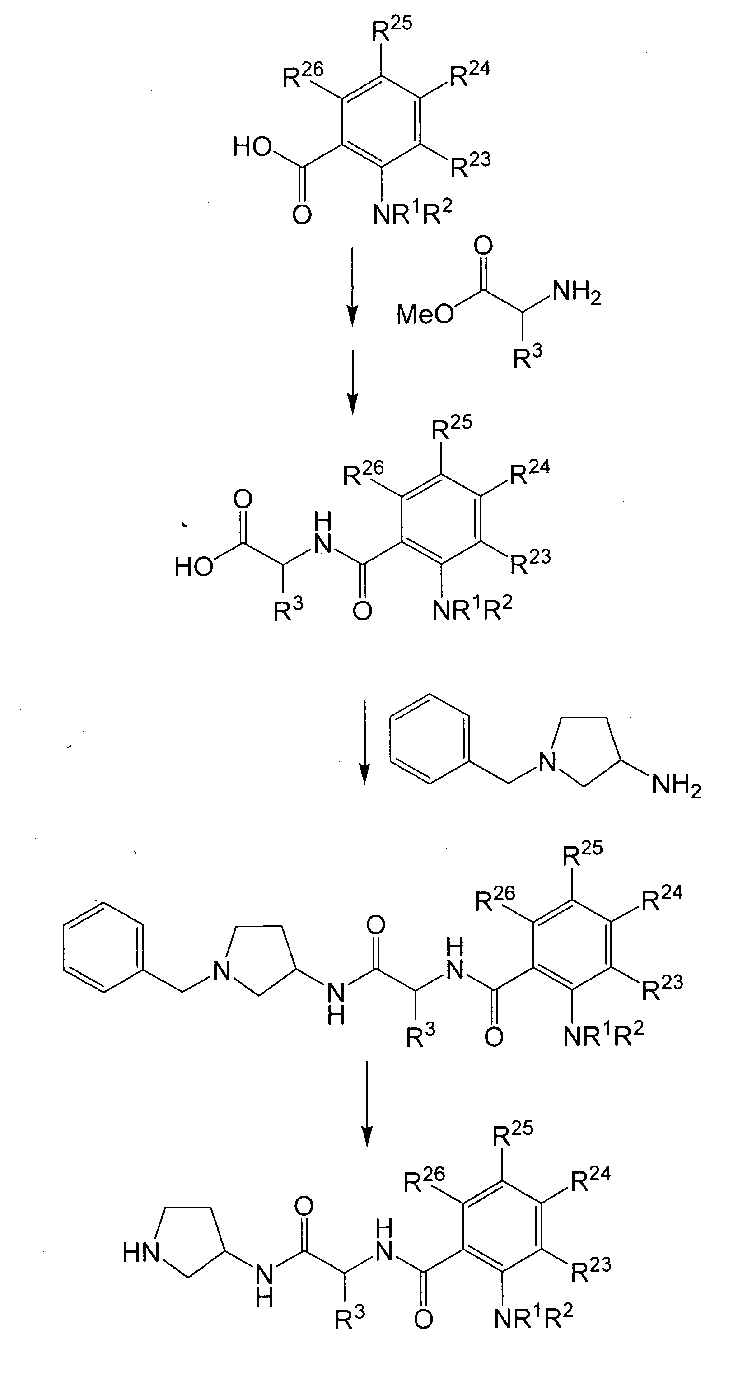

Namely, an anthranilic acid having an amino group at the 2-position is successively condensed with an amino acid and then with benzylaminopyrrolidine. Removal of the protecting benzyl group from the aminopyrrolidine derivative thus obtained gives the starting material necessary for the present invention.

However, acquisition of raw materials used in the present invention is not limited to the synthetic route exemplified here. Furthermore, the production method exemplified here illustrates outline of the synthetic route. Any optional steps such as utilization of protecting groups may be added depending on compounds and conditions employed in each reaction.

The second invention is explained below. Here R³, R¹¹, R¹², R¹⁴, R¹⁵, R¹⁶, R¹⁷, R²³, R²⁴, R²⁵ and R²⁶ in the formula are as defined above. R⁵ and R⁶ represent independently hydrogen or a protecting group for amino group (wherein R⁵ and R⁶ may, taken together, form a cyclic structure) with the proviso that both R⁵ and R⁶ are not simultaneously hydrogen.

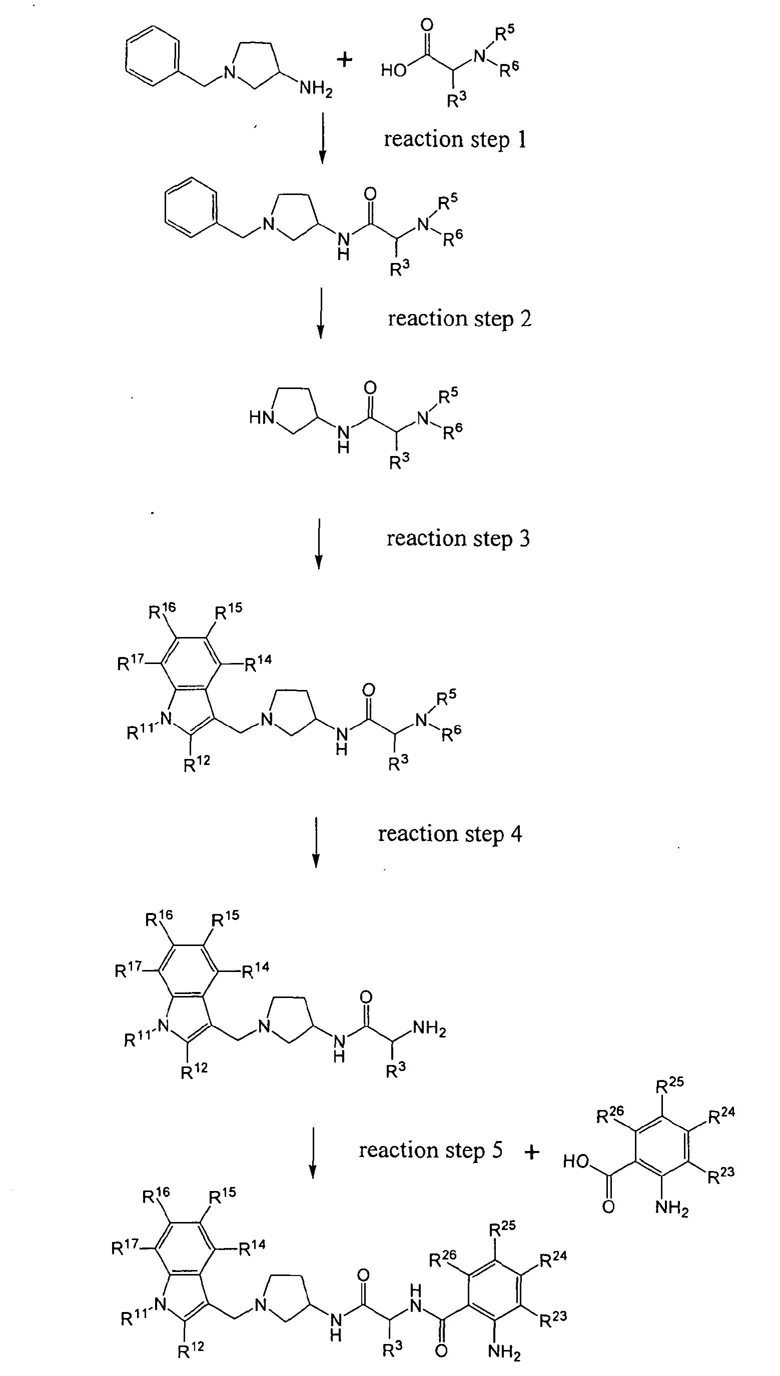

Reaction step 5 in the above formula is condensation reaction, which proceeds by treatment with an anthranilic acid derivative in the presence of a condensing reagent in organic solvent.

As organic solvents there may be mentioned *N,N*-dimethylformamide, tetrahydrofuran, acetate esters, toluene, acetonitrile, dimethoxyethane, chloroform, dichloromethane, methanol and 2-propanol. Among these, from the viewpoint of industrial production method, tetrahydrofuran, *N*,*N*-dimethylformamide, ethyl acetate, 2-propanol and methanol are preferred. Tetrahydrofuran, ethyl acetate, and a mixed solvent of one of these with 2-propanol or methanol are especially preferred.

As a condensing reagent there may be mentioned 1,3-dicyclohexylcarbodiimide, isobutyl chloroformate, pivaloyl chloride, isovaleryl chloride, 1-ethyl-3-(3-dimethylaminopropyl)carbodiimide, 1-cyclohexyl-3-morpholinoethylcarbodiimide, 1-cyclohexyl-3-(4-diethylaminocyclohexyl)carboximide, *N,N*'-carbonyldiimidazole and 2-chloro-1,3-dimethylimidazolinium chloride. In particular, 1-ethyl-3-(3-dimethylaminopropyl)carbodiimide, 1-cyclohexyl-3-morpholinoethylcarbodiimide and 1-cyclohexyl-3-(4-diethylaminocyclohexyl)carboximide are preferred. 1-Ethyl-3-(3-dimethylaminopropyl)carbodiimide hydrochloride is especially preferred. Carbodiimide reagents in the present specification include their hydrochloride.

In this reaction, it is preferred to use an additive for accelerating the reaction or increasing the yield. As such an additive there may be mentioned *p*-nitrophenol, hydroxysuccinimide, hydroxyphthalimide, 1-hydroxy-1,2,3-benzotriazole, 3-hydroxy-4-oxo-3,4-dihydroxy-1,2,3-benzotriazine, *N*-hydroxy-5-norbornene-2,3-dicarboximide and ethyl 2-hydroxyimino-2-cyanoacetate. In particular hydroxysuccinimide and 1-hydroxy-1,2,3-benzotriazole are preferred. Such an additive is usually added at 0.1-1.0 equivalent. Addition of 0.1-0.2 equivalent of 1-hydroxy-1,2,3-benzotriazole is more preferred.

Furthermore, when an amine hydrochloride is used in amidation, it is preferred to add a base to accelerate the reaction by removal of hydrogen chloride formed. Such a base can be used together with the additive described above. As a base, triethylamine and N-methylmorpholine can be used. Triethylamine is particularly preferred.

Namely, the procedure which is the most convenient to handle and particularly preferred is to carry out the reaction using tetrahydrofuran or ethyl acetate as a solvent in the presence of 1-ethyl-3-(3-dimethylaminopropyl)carbodiimide hydrochloride, 1-hydroxy-1,2,3-benzotriazole and triethylamine.

The starting material used in the reaction step 5 described above is preferably synthesized by reaction step 4 in the above reaction formula. Here, as preferred protecting group(s) for amino group as R⁵ and R⁶ there may be mentioned methoxycarbonyl, *t*-butoxycarbonyl, benzyloxycarbonyl, allyloxycarbonyl, formyl, acetyl, benzoyl, methyl, ethyl, allyl, benzenesulfonyl and phthaloyl. When the protecting group(s) contain aromatic ring(s), the aromatic ring(s) may be substituted with one or more of nitro, amino, C₁-C₆ alkyl, C₁-C₆ alkoxy or halogen. In particular, hydrogen and *t*-butoxycarbonyl are preferred as R⁵ and R⁶.

Reaction step 4 is reaction for deprotecting an amino group. For example, when *t*-butoxycarbonyl is used as the protecting group for amino group, it can be removed by addition of an acid such as hydrochloric acid, hydrogen chloride solution such as hydrogen chloride/diethyl ether, hydrogen chloride/1,4-dioxane, hydrogen chloride/methanol and the like, or acid such as trifluoroacetic acid and the like in organic solvent. Use of hydrogen chloride/1,4-dioxane or hydrogen chloride/methanol solution is especially preferred.

The starting material used in reaction step 4 is preferably synthesized by reaction step 3 in the above reaction formula.

Reaction step 3 is reaction for introducing an indole derivative into an aminopyrrolidine skeleton. This step is similar to reaction step 1 in the first invention and largely classified to two methods. One of them is reaction of an indole derivative having no substituent at the 3-position with an amine in the presence of a synthon of formaldehyde such as formalin, paraformaldehyde and trioxane. Namely, in solvent such as acetic acid/1,4-dioxane (mixing ratio is for example 1:1), ethanol, methanol and acetic acid/methanol (with any mixing ratio), reaction where the 3-position in the indole derivative and the primary or secondary amine are bonded via one methylene group proceeds. It is preferred to carry out the reaction in ethanol at room temperature. As a synthon of formaldehyde, 37% formalin is particularly preferred.

Alternatively, an indole derivative having a dialkylaminomethyl group at the 3-position may be used. Namely, reaction of an indole derivative with an amine in organic solvent gives the desired indole derivative. Preferred solvents are alcohols and organic solvents with boiling points of 70-120°C. 2-Propanol and toluene are especially preferred. The crude product thus obtained may be converted to a salt such as monohydrochloride for purification.

The staring material used in reaction step 3 is preferably synthesized by reaction step 2 in the above formula.

Reaction step 2 is reductive reaction to remove a benzyl group. An example of this reaction is reductive debenzylation in the presence of palladium catalyst such as Pd/C and palladium(II) hydroxide on carbon in alcoholic solvent such as methanol, ethanol and 2-propanol using hydrogen source such as gaseous hydrogen, formic acid and ammonium formate. To obtain the product as a free base, gaseous hydrogen is preferred as hydrogen source.

The starting material used in reaction step 2 is preferably synthesized by reaction step 1 in the above reaction formula.

Reaction step 1 is condensation reaction similar to reaction step 5. The reaction proceeds by treatment with an amino-group-protected amino acid using a condensing agent in organic solvent. Preferred reaction conditions are the same as described for reaction step 5.

The final product in the second invention, an aminopyrrolidine derivative, and their synthetic intermediates contain a basic nitrogen and may form a salt. As an example of such a salt there may be mentioned hydrochloride, sulfate, acetate, phosphate, citrate and the like. In particular hydrochloride, sulfate and acetate are preferred.

The final product, an aminopyrrolidine derivative, contains a chiral carbon and enantiomers may exist. According to the method of the present invention, either of the enantiomers and a mixture thereof can be produced.

The third and fourth inventions are explained below.

In the formula representing compounds of the third invention or their synthetic methods of the fourth invention, R²³, R²⁴, R²⁵ and R²⁶ represent independently hydrogen, halogen, optionally halogenated C₁-C₆ alkyl, optionally halogenated C₁-C₆ alkoxy or hydroxyl. Preferably R²³, R²⁴ and R²⁶ are all hydrogen. On the other hand, R²⁵ is preferably halogenated C₁-C₆ alkoxy, especially preferably trifluoromethoxy.

Because this compound contains a basic nitrogen and may also contain a carboxyl group, a salt may be formed with either an acid or a base, if chemically possible.

Among the production methods of the present invention described above, a method via the following reaction steps is preferred. Here, R¹, R², R³, R⁴, R²³, R²⁴, R²⁵ and R²⁶ in the formula are as defined above.

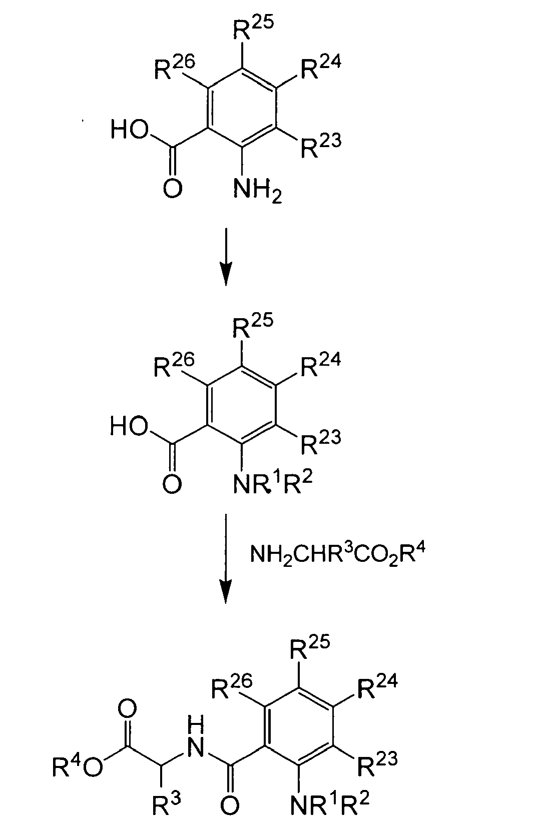

In the following, the method for producing the compound of the present invention is explained with representative examples. Other compounds of the present invention represented by the above formula can also be produced with reference to these examples. From viewpoints of product yields, production costs, purity and the like, it is preferred to optimize reaction conditions such as solvent, reaction temperature, reaction time and substrate concentration beforehand depending on desired compounds. The optimization can be easily performed by those skilled in the art with reference to the present specification, particularly the examples, although it is not indispensable in carrying out the present invention.

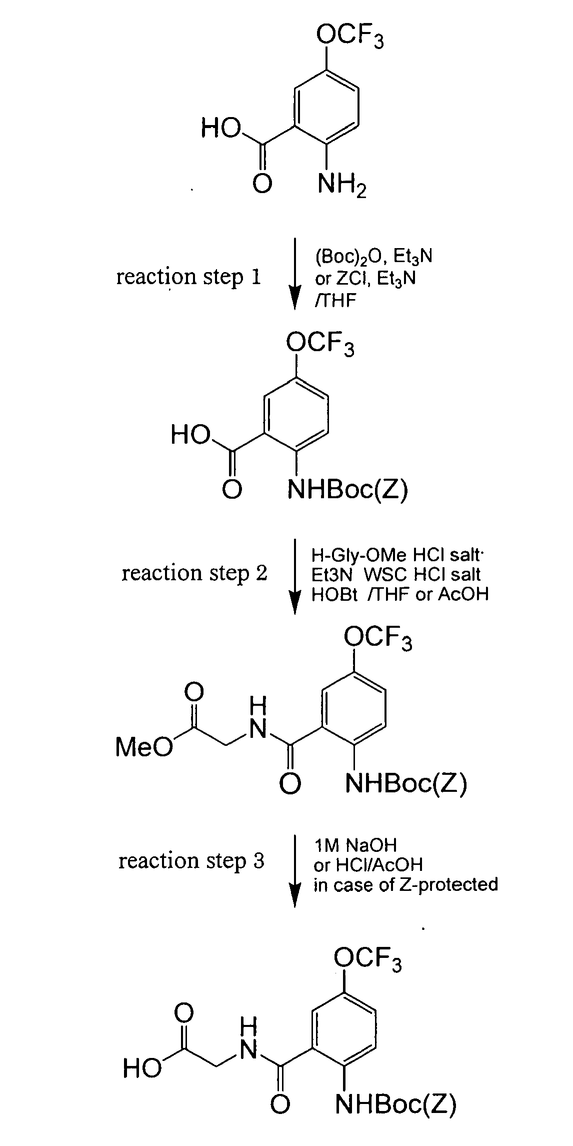

Reaction step 1 in the above formula is a step for introducing a protective group of the amino group in anthranilic acid. A protecting group that forms carbamate with said amino group, for example *t*-butoxycarbonyl or benzyloxycarbonyl, is preferred. Other specific examples were described above as preferred groups for R¹ and R². For a step introducing a protecting group, an amino group is treated with a reagent such as (Boc)₂O and Z-Cl. Here, *N,N*-dimethylformamide, tetrahydrofuran, acetate ester or the like is employed as a reaction solvent. It is preferred to add a tertiary amine such as triethylamine and pyridine for accelerating the reaction, although the reaction proceeds without such an additive.

Even if the subsequent reaction is carried out without protection, the desired amide can be produced, but it is problematic that a cyclocondensation product is formed as a byproduct upon hydrolysis in reaction step 3.

Reaction step 2 is condensation reaction, which proceeds by treatment with a carboxy-protected amino acid or its salt in the presence of a condensing agent in organic solvent.

*N,N-*dimethylformamide, tetrahydrofuran, ethyl acetate, toluene, acetonitrile, dimethoxyethane, chloroform, dichloromethane, methanol or 2-propanol is employed as reaction solvent. Considering industrial use, preferred solvents are tetrahydrofuran, *N*,*N*-dimethylformamide, acetate esters, methanol and 2-propanol, and tetrahydrofuran and ethyl acetate are especially preferred.

As a condensing agent can be used 1,3-dicyclohexylcarbodiimide, isobutyl chloroformate, pivaloyl chloride, isovaleryl chloride, 1-ethyl-3-(3-dimethylaminopropyl)carbodiimide or hydrochloride thereof, 1-cyclohexyl-3-morpholinoethylcarbodiimide, 1-cyclohexyl-3-(4-diethylaminocyclohexyl)carboximide, *N*,*N*'-carbonyldiimidazole or , 2-chloro-1,3-dimethylimidazolinium chloride. Among them, 1-ethyl-3-(3-dimethylaminopropyl)carbodiimide, 1-cyclohexyl-3-morpholinoethylcarbodiimide, and 1-cyclohexyl-3-(4-diethylaminocyclohxyl)carboximide are preferred, and 1-ethyl-3-(3-dimethylaminopropyl)carbodiimide hydrochloride is especially preferred.

In this step, it is preferred to use an additive for accelerating the reaction or increasing the yield. As an additive can be used p-nitrophenol, hydroxysuccinimide, hydroxyphthalimide, 1-hydroxy-1,2,3-benzotriazole, 3-hydroxy-4-oxo-3,4-dihydro-1,2,3-benzotriazine, *N*-hydroxy-5-norbornene-2,3-dicarboximide or ethyl 2-hydroxyimino-2-cyanoacetate. In particular, hydroxysuccinimide or 1-hydroxy-1,2,3-benzotriazole is preferred. Such an additive is usually added at 0.1-1.0 equivalent. It is more preferred to add 0.1-0.2 equivalent of 1-hydroxy-1,2,3-benzotriazole.

When an amine hydrochloride is used in amidation, it is preferred to add a base to accelerate the reaction by removing hydrogen chloride formed. The base can be used together with the additive described above. As a base there may be mentioned triethylamine and N-methylmorpholine, and triethylamine is particularly preferred.

Reaction step 3, which is optional, is a step for deprotecting the carboxyl group of the amino acid. For protecting a carboxyl group of an amino acid, it is preferred to form an ester especially methyl ester. In this case the ester is hydrolyzed either with alkaline in methanol, tetrahydrofuran, water or a mixture thereof or with concentrated hydrochloric acid in tetrahydrofuran or acetic acid. In either case the reaction is carried out under usual hydrolytic conditions.

### EXAMPLES

The present invention will be explained in more detail with examples below, although the present invention is not limited thereto.

### Example 1

### Synthesis of 2-(2-benzyloxycarbonylamino)-5-trifluoromethoxybenzoic acid

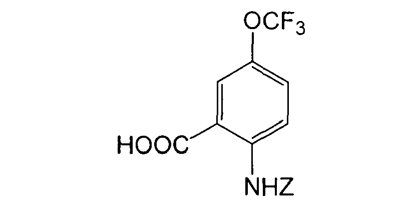

2-Amino-5-triflurormethoxybenzoic acid (21.1 g) was dissolved in ethyl acetate (100 mL). Here were added triethylamine (2.02 g) and then benzyloxycarbonylchloride (18.76 g). After the mixture was kept at 30°C for 2 hr, ethyl acetate (100 mL) and water (100 mL) were added. The organic layer was separated, washed with water (200 mL) and 10% aqueous citric acid (200 mL), dried over anhydrous sodium sulfate and filtered to remove the drying agent. The organic solvent was concentrated to obtain the title compound (24.66 g).
¹H NMR (200 MHz, DMSO-*d*_{*6*}, relative to TMS); δ 5.20 (s, 2H), 7.35-7.56 (m, 5H), 7.63-7.80 (m, 1H), 7.83-7.96 (m, 1H), 8.36-8.56 (m, 1H), 10.78 (s, 1H).

### Example 2

### Synthesis of methyl 2-(2-t-butoxycarbonylamino-5-trifluoromethoxybenzamido)acetate

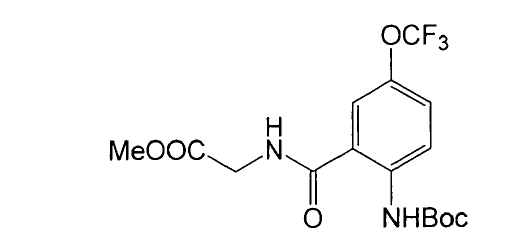

2-*t*-Butoxycarbonylamino-5-trifluoromethoxybenzoic acid (96.38 g) was dissolved in toluene (2000 mL). Here were added glycine methyl ester hydrochloride (45.20 g), triethylamine (35.43 g) and 1-hydroxy-1,2,3-benzotriazole hydrate (9.19 g), and then 1-ethyl-3-(3-dimethylaminopropyl)carbodiimide hydrochloride (69.01 g). The mixture was kept at 30°C for reaction for 7 hr. The solvent was removed from the reaction mixture under reduced pressure, and ethyl acetate (2000 mL) and water (2000 mL) were added to the residue. The organic layer was separated, washed with 10% aqueous citric acid (2000 mL) and water (2000 mL) and dried over anhydrous sodium sulfate. The organic solvent was concentrated to obtain the title compound (113.64 g).
¹H NMR (200 MHz, DMSO-d_{*6*}, relative to TMS); δ 1.47 (s, 9H), 3.69 (s, 3H), 4.04 (d, *J*= 5.5 Hz, 2H), 4.35 (brs, 1H), 7.57 (dd, *J =* 9.2 and 2.6 Hz, 1H), 7.80 (d, *J*= 2.6 Hz, 1H), 8.35 (d, *J* = 9.2 Hz, 1H), 9.39 (t, *J=* 5.7 Hz, 1H), 10.64 (s, 1H).

### Example 3

### Synthesis of 2-(2-t-butoxycarbonylamino-5-trifluoromethoxybenzamido)acetic acid

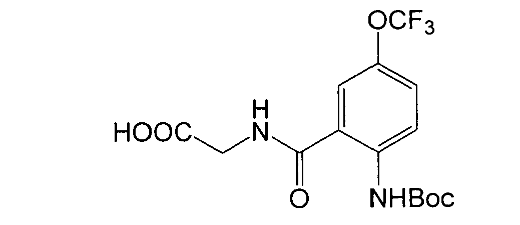

Methyl 2-(2-*t*-butoxycarbonylamino-5-trifluoromethoxybenzamido)acetate (113.64 g) was dissolved in a mixture of tetrahydrofuran (300 mL) and methanol (300 mL). Here 1 M aqueous sodium hydroxide (347.6 mL) was added with ice bath cooling. The mixture was kept with ice bath cooling for 1 hr, diluted with ethyl acetate (3000 mL) and neutralized with 10% aqueous citric acid (1500 mL). The organic layer was separated, washed with 10 % aqueous sodium chloride (1500 mL) twice and dried over anhydrous sodium sulfate. The organic solvent was distilled off to obtain the title compound (113.70 g). This crude product was dissolved in hexane/ethanol (40/3; 1935 mL) with heating and the solution was cooled to room temperature and then further ice-cooled. Needle-like crystals precipitated were filtered to obtain the title compound (80.27 g).
¹H NMR (200 MHz, DMSO-*d*₆, relative to TMS); δ 1.47 (s, 9H), 3.82 (d, *J*= 5.7 Hz, 2H), 4.35 (brs, 1H), 7.54 (dd, *J =* 9.2 and 2.6 Hz, 1H), 7.78 (d, *J =* 2.6 Hz, 1H), 8.34 (d, *J* = 9.2 Hz, 1H), 9.28 (t, *J=* 5.7 Hz, 1H), 10.68 (s, 1H).

### Example 4

### Synthesis of methyl 2-(2-benzyloxycarbonylamino-5-trifluoromethoxybenzamido)acetate

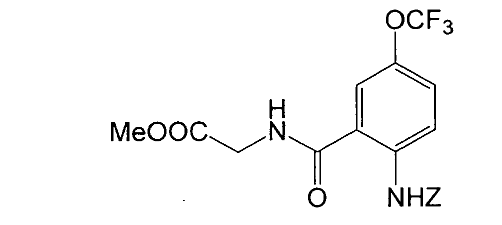

2-Benzyloxycarbonylamino-5-trifluoromethoxybenzoic acid (50.0 g) was dissolved in tetrahydrofuran (400 mL). Here were added glycine methyl ester hydrochloride (8.81 g), triethylamine (15.18 g), 1-hydroxy-1,2,3-benzotriazole hydrate (9.46 g), and then 1-ethyl-3-(3-dimethylaminopropyl)carbodiimide hydrochloride(13.42 g). After the mixture was kept at 30°C for reaction for 3 hr, the solvent was distilled off. To the residue were added ethyl acetate (300 mL) and water (300 mL). The organic layer was separated, washed with water (300 mL) and saturated aqueous sodium hydrogencarbonate (400 mL), dried over anhydrous sodium sulfate, filtered to remove the drying agent, and concentrated to obtain the title compound (38.53 g).
¹H NMR (200 MHz, DMSO-*d*₆, relative to TMS); δ 3.67 (s, 3H), 4.04 (d, *J=* 5.4 Hz, 2H), 5.18 (s, 2H), 7.34-7.48 (m, 5H), 7.58-7.64 (m, 1H), 7.80-7.88 (m, 1H), 8.31-8.36 (m, 1H), 9.47 (t, *J=* 5.4 Hz, 1H)

### Example 5

### Synthesis of 2-(2-benzyloxycarbonylamino-5-trifluoromethoxybenzamido)acetic acid

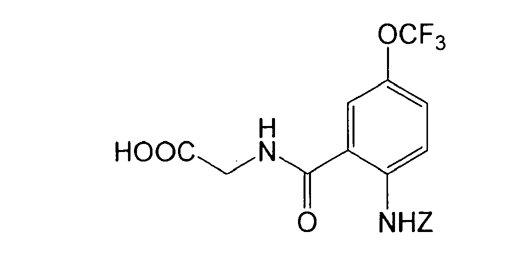

Methyl 2-(2-benzyloxycarbonylamino-5-trifluoromethoxybenzamido)acetate (10.0 g) was dissolved in tetrahydrofuran (40 mL). Here were added concentrated hydrochloric acid (19.9 mL) and acetic acid (40 mL), and the mixture was kept at 30°C for reaction overnight. After distilling off the solvent, ethyl acetate (200 mL) and water (200 mL) were added to the residue. The organic layer was separated, washed with saturated aqueous sodium hydrogencarbonate (400 mL) and 10% aqueous citric acid (200 mL), dried over anhydrous sodium sulfate, filtered to remove the drying agent, and concentrated to obtain the title compound (7.90 g).
¹H NMR (200 MHz, DMSO-*d*_{*6*}, relative to TMS); δ 3.93 (d, *J*= 5.4 Hz, 2H), 5.17 (s, 2H), 7.38-7.50 (m, 5H), 7.54-7.68 (m, 1H), 7.78-7.84 (m, 1H), 8.31-8.36 (m, 1H), 9.30-9.38 (m, 1H), 10.95 (s, 1H).

### Example 6

### Synthesis of methyl 2-(2-amino-5-trifluoromethoxybenzamido)acetate

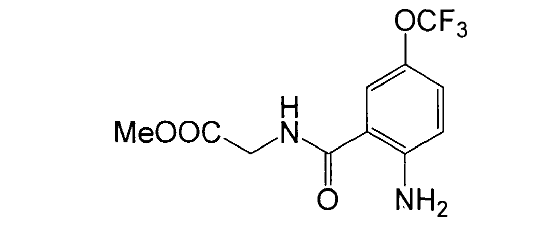

2-Amino-5-trifluoromethoxybenzoic acid (44.23 g) was dissolved in tetrahydrofuran (1000 mL). Here were added glycine methyl ester hydrochloride (27.62 g), triethylamine (22.26 g), 1-hydroxy-1,2,3-benzotriazole hydrate (33.69 g) and then 1-ethyl-3-(3-dimethylaminopropyl)carbodiimide hydrochloride (42.17 g). The mixture was kept at 30°C for reaction for 6.5 hr. The solvent was distilled off under reduced pressure, and ethyl acetate (1000 mL) and water (1000 mL) were added to the residue. The organic layer was separated, washed with 5% aqueous sodium hydrogencarbonate (1000 mL) twice, dried over anhydrous sodium sulfate, and concentrated to obtain the title compound (62.03 g).
¹H NMR (200 MHz, DMSO-*d*₆, relative to TMS); δ 3.67 (s, 3H), 3.96 (d, *J* = 5.7 Hz, 2H), 6.73 (brs, 2H), 6.79 (d, *J* = 9.0 Hz, 1H), 7.20 (dd, *J =* 9.0 and 2.7 Hz, 1H), 7.56 (d, *J =* 2.7 Hz, 1H), 8.85 (t, *J=* 5.7 Hz, 1H).

### Example 7

### Synthesis of 2-(2-amino-5-trifluoromethoxybenzamido)acetic acid

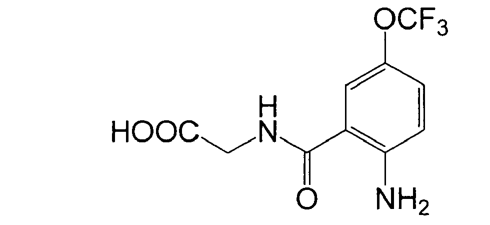

Methyl 2-(2-amino-5-trifluoromethoxybenzamido)acetate (43.83 g) was dissolved in a mixture of tetrahydrofuran (150 mL) and methanol (150 mL). Here 1 M aqueous sodium hydroxide (180 mL) was added with ice bath cooling. The mixture was kept with ice bath cooling for reaction for 0.5 hr, diluted with ethyl acetate (1500 mL) and neutralized with 10% aqueous citric acid (1500 mL). The organic layer was separated, washed with water (1500 mL) twice and dried over anhydrous sodium sulfate. The organic solvent was distilled off to obtain the title compound (40.51 g).
¹H NMR (200 MHz, DMSO-*d*₆, relative to TMS); δ 3.86 (d, *J*= 5.9 Hz, 2H), 6.71 (brs, 1H), 6.77 (d, *J=* 10.0 Hz, 1H), 7.19 (dd, *J* = 10.0 and 2.1 Hz, 1H), 7.55 (d, *J=* 2.1 Hz, 1H), 8.74 (t, *J* = 5.9 Hz, 1H), 12.61 (s, 1H).

### Example 8

### Synthesis of (R)-3-[2-(2-t-butoxycarbonylamino-5-trifluoromethoxybenzamido)acetamido] -1-(6-methylindol-3-ylmethyl)pyrrolidine

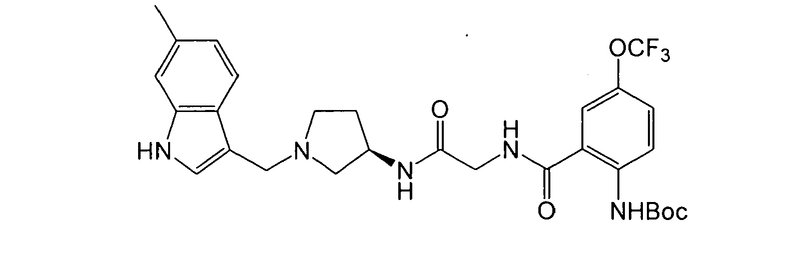

(*R*)-3-[2-(2-*t*-butoxycarbonylamino-5-trifluoromethoxybenzamido)acetamido]pyrrolidine (5.07 g) and 6-methylgramine (gramine is 3-dimethylaminomethylindole; 1.98 g) were dissolved in 2-propanol (100 mL). From this solution the solvent was distilled off at 95°C under slightly reduced pressure with stirring. To the residue 2-propanol was newly added and the solvent was again distilled off under slightly reduced pressure. This procedure was repeated five times. Ethyl acetate (100 mL) was added to the residue obtained finally, and this organic solution was washed with 1 M aqueous sodium hydroxide (100 mL) and then saturated brine (100 mL) twice, dried over anhydrous sodium sulfate, filtered to remove the drying agent, and distilled off under reduced pressure to obtain the title compound (6.07 g).
¹H NMR (200 MHz, DMSO-d_{*6*}, relative to TMS); δ 1.46 (s, 9H), 1.48-1.63 (m, 1H), 1.99 -2.16 (m, 1H), 2.25-2.44 (m, 2H), 2.37 (s, 3H), 2.56-2.71 (m, 2H), 3.68 (s, 2H), 3.80 (d, *J*= 5.5 Hz, 2H), 4.05-4.22 (m, 1H), 6.79 (d, *J* = 8.4 Hz, 1H), 7.09-7.12 (m, 2H), 7.46 (d, *J* = 8.1 Hz, 1H), 7.51-7.56 (m, 1H), 7.75 (d, *J* = 2.6 Hz, 1H), 8.13 (d, *J* = 7.0 Hz, 1H), 8.30 (d, *J* = 9.3 Hz, 1H), 9.05 (t, *J =* 5.5 Hz, 1H), 10.63 (brs, 1H), 10.73 (brs, 1 H).

### Example 9

### Synthesis of (R)-3-[2-(2-amino-5-trifluoromethoxybenzamido)acetamido]-1-(6-methylindol-3-ylmethyl)pyrrolidine

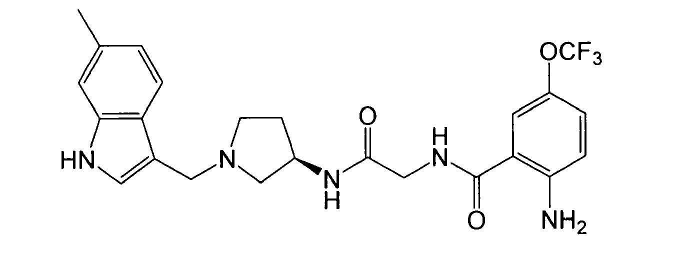

A 10% hydrogen chloride/methanol solution (30 mL) was added to (*R*)-3-[2-(2-*t*-butoxycarbonylamino-5-trifluoromethoxybenzamido)acetamido]-1-(6-methylindol-3-ylmethyl)pyrrolidine (6.07 g), and the mixture was stirred at 50°C for 2 hr. To the reaction mixture were added 2 M hydrochloric acid (120 mL) and ethyl acetate (120 mL). The aqueous layer was separated, neutralized with 2 M aqueous sodium hydroxide (300 mL) and the resultant solution was extracted with ethyl acetate (120 mL). The organic extract was washed with saturated brine (120 mL) twice, dried over anhydrous sodium sulfate, filtered to remove the drying agent, and the organic solvent was distilled off under reduce pressure to obtain the crude product, which was further purified to obtain the title compound (3.49 g).
¹H NMR (200 MHz, DMSO-d_{*6*}, relative to TMS); δ 1.48-1.62 (m, 1H), 1.99-2.15 (m, 1H), 2.28-2.46 (m, 2H), 2.37 (s, 3H), 2.56-2.69 (m, 2H), 3.33 (s, 2H), 3.75 (d, *J=* 5.9 Hz, 2H), 4.06-4.22 (m, 1H), 6.64 (brs, 2H), 6.76 (d, *J* = 9.2 Hz, 1H), 6.79 (d, *J =* 8.1 Hz, 1H), 7.12-7.19 (m, 3H), 7.47 (d, *J =* 8.1 Hz, 1H), 7.52 (d, *J =* 2.9 Hz, 1H), 8.04 (d, *J* = 7.3 Hz, 1 H), 8.51 (t, *J=* 5.9 Hz, 1H), 10.73 (brs, 1H).

### Example 10

### Synthesis of (R)-3-[2-(2-amino-5-trifluoromethoxybenzamido)acetamido]-1-(6-methylindol-3-ylmethyl)pyrrolidine

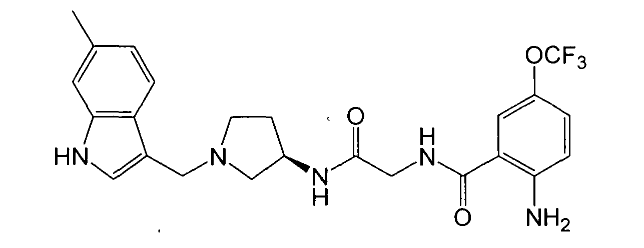

(*R*)-3-[2-(2-Amino-5-trifluoromethoxybenzamido)acetamido]pyrrolidine monoacetate (3.00 g) and 6-methylgramine (1.46 g) were dissolved in 2-propanol (50 mL). From this solution the solvent was distilled off under slightly reduced pressure at 95°C with stirring. To the residue 2-propanol was newly added and the solvent was again distilled off under slightly reduced pressure. This procedure was repeated seven times. To the residue (4.20 g) obtained finally, ethyl acetate (42 mL) was added, and the mixture was extracted with 1 M hydrochloric acid (84 mL). The organic layer was washed with 1 M hydrochloric acid. The combined aqueous extracts were neutralized (pH =9-10) with 1 M aqueous sodium hydroxide (128 mL) and the resultant solution was extracted with ethyl acetate (42 mL). The organic extract was washed with 10% aqueous sodium chloride (50 mL) twice. The organic solvent was distilled off under reduced pressure to obtain the crude product, which was further purified to obtain the title compound (0.50 g).
¹H NMR (200 MHz, DMSO-d₆, relative to TMS); δ 1.48-1.62 (m, 1H), 1.99-2.15 (m, 1H), 2.28-2.46 (m, 2H), 2.37 (s, 3H), 2.56-2.69 (m, 2H), 3.33 (s, 2H), 3.75 (d, *J*= 5.9 Hz, 2H), 4.06-4.22 (m, 1H), 6.64 (brs, 2H), 6.76 (d, *J* = 9.2 Hz, 1H), 6.79 (d, *J* = 8.1 Hz, 1H), 7.12-7.19 (m, 3H), 7.47 (d, *J* = 8.1 Hz, 1H), 7.52 (d, *J* = 2.9 Hz, 1H), 8.04 (d, *J* = 7.3 Hz, 1H), 8.51 (t, *J* = 5.9 Hz, 1H), 10.73 (brs, 1H).

### Example 11

### Synthesis of (R)-3-[2-(2-amino-5-trifluoromethoxybenzamido)acetamido]-1-(6-methylindol -3-ylmethyl)pyrrolidine

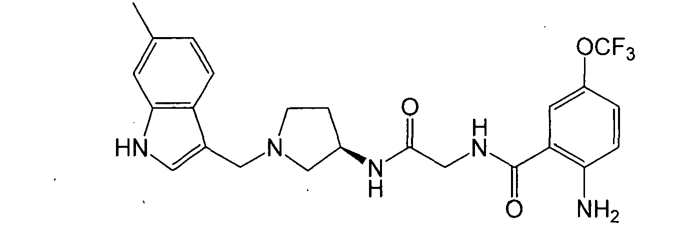

(*R*)-3-[2-(2-Amino-5-trifluoromethoxybenzamido)acetamido]-1-benzylpyrrolidine hydrochloride (6.16 g) and 6-methylgramine (3.03 g) were dissolved in 2-propanol. Here triethylamine (1.79 g) was added, and the solution was kept at 80°C for reaction for 7 hr. The solvent was distilled off, and ethyl acetate (200 mL) and water (200 mL) were added to the residue. The organic layer was separated and extracted with 2 M hydrochloric acid (120 mL). Ethanol (240 mL) was added to this aqueous layer to yield a homogeneous solution, which was neutralized with 2 M aqueous sodium hydroxide (120 mL). The resultant insoluble material was filtered and dried under reduced pressure to obtain the title compound (1.70 g).
¹H NMR (200 MHz, DMSO-d_{*6*}, relative to TMS); δ 1.48-1.62 (m, 1H), 1.99-2.15 (m, 1H), 2.28-2.46 (m, 2H), 2.37 (s, 3H), 2.56-2.69 (m, 2H), 3.33 (s, 2H), 3.75 (d, *J* = 5.9 Hz, 2H), 4.06-4.22 (m, 1H), 6.64 (brs, 2H), 6.76 (d, *J* = 9.2 Hz, 1H), 6.79 (d, *J =* 8.1 Hz, 1H), 7.12 -7.19 (m, 3H), 7.47 (d, *J* = 8.1 Hz, 1H), 7.52 (d, *J=* 2.9 Hz, 1H), 8.04 (d, *J* = 7.3 Hz, 1H), 8.51 (d, *J=* 5.9 Hz, 1H), 10.73 (brs, 1H).

### Example 12

### Synthesis of (R)-2-(t-butoxycarbonylamino)-N-(1-benzylpyrrolidin-3-yl)acetamide

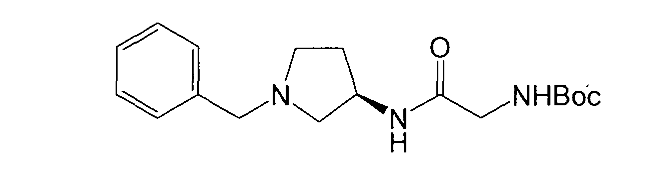

(*R*)-1-Benzyl-3-aminopyrrolidine (100.6 g) was dissolved in tetrahydrofuran (2000 mL). Here were added *N*-Boc-glycine (100.0 g), 1-hydroxy-1,2,3-benzotriazole (81.2 g), 1-ethyl-3-(3-dimethylaminopropyl)carbodiimide hydrochloride (114.5 g) and triethylamine (84 mL). The resultant mixture was stirred at 30°C for 3 hr. After insoluble material was filtered off, the solvent was distilled off under reduced pressure. To the residue were added water (800 mL) and ethyl acetate (1000 mL). The organic layer was separated and washed with saturated aqueous sodium hydrogencarbonate (800 mL) and then saturated brine (800 mL). The combined aqueous layers were further extracted with ethyl acetate (800 mL). All the organic layers were combined, dried over anhydrous magnesium sulfate, filtered to remove the drying agent, and concentrated under reduced pressure to obtain the title compound (179.9 g).
¹H NMR (400 MHz, CHCl₃-*d*); δ 1.45 (s, 9H), 1.57-1.64 (m, 1H), 2.24-2.32 (m, 2H), 2.52- 2.60 (m, 2H), 2.82-2.89 (m, 1H), 3.60 (s, 2H), 3.73 (d, *J* = 6.9 Hz, 2H), 4.43-4.47 (m, 1H), 5.18 (s, 1H), 6.44 (d, *J* = 6.9 Hz, 1H), 7.23-7.35 (m, 5H).

### Example 13

### Synthesis of (R)-2-(t-butoxycarbonylamino)-N-pyrrolidin-3-ylacetamide

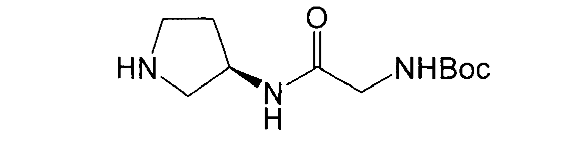

Under an argon atmosphere (*R*)-2-(*t*-butoxycarbonylamino)-*N*-(1-benzylpyrrrolidin-3-yl)acetamide (169.0 g) was dissolved in ethanol (800 mL). Here 10% Pd/C (24.0 g; dry weight) moistened with ethanol was added, and the mixture was stirred at 60°C overnight under a stream of hydrogen gas. The reaction mixture was filtered through a celite pad. The filtrate was concentrated under reduced pressure to obtain the crude product, which was crystallized from ethyl acetate (150 mL) to obtain the title compound (94.7g).
¹H NMR (400 MHz, DMSO-*d*₆); δ 1.35 (s, 9H), 1.42-1.51 (m, 1H), 1.81-1.90 (m, 1H), 2.46-2.50 (m, 1H), 2.67-2.75 (m, 1H), 2.78-2.98 (m, 2H), 3.46 (s, 1H), 3.47 (s, 1H), 3.50 (s, 1H), 4.06 (d, *J* = 5.4 Hz), 6.85 (t, *J* = 5.6 Hz, 1H), 7.84 (d, *J* = 7.1 Hz, 1H).

### Example 14

Except for using 20% palladium (II) hydroxide on carbon (50% w/w hydrated form; 620 mg) instead of 10% Pd/C (24.0 g; dry weight) moistened with ethanol and a reaction temperature of 20°C instead of 60°C, the same procedure as Example 13 was carried out to obtain (*R*)-2-(*t*-butoxycarbonylamino)-*N*-pyrrolidin-3-ylacetamide (2.3 g).

### Example 15

### Synthesis of (R)-2-(t-butoxycarbonylamino)-N-[1-(indol-3-ylmethyl)pyrrolidin-3-yl]acetamide

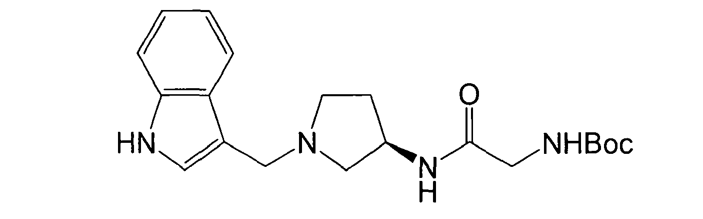

To (*R*)-2-(*t*-butoxycarbonylamino)-*N*-pyrrolidin-3-ylacetamide(1.96 g) were added a solution of acetic acid/1,4-dioxane (1:1 mixed solvent; 20 mL) containing indole (1.06 g) and then 37% formalin (727 µL). The resultant solution was stirred at room temperature overnight, concentrated under reduced pressure and diluted with ethyl acetate (50 mL). Extraction was performed after neutralizing the solution (pH =10) with 1 M aqueous sodium hydroxide. The organic layer was separated, and the aqueous layer was further extracted with ethyl acetate (50 mL). The combined organic layers were washed with saturated brine (50 mL) and dried over anhydrous sodium sulfate. After filtering , the filtrate was concentrated under reduced pressure to obtain the title compound (2.75 g) as brown amorphous solid.
¹H NMR (400MHz, DMSO-*d*_{*6*}); δ 1.35 (s, 9H), 1.49-1.51 (m, 1H), 1.98-2.12 (m, 1H), 2.27-2.44 (m, 2H), 2.57-2.67 (m, 2H), 3.31 (s, 2H), 3.70 (s, 2H), 4.06-4.18 (m, 1H), 4.59 (s, 1H), 6.79 (s, 1H), 6.93-7.07 (m, 2H), 7.80 (d, *J =* 7.4 Hz, 1H), 7.59 (d, *J* = 7.4 Hz, 1H), 10.88 (s, 1H).

### Example 16

### Synthesis of (R)-2-(t-butoxycarbonylamino)-N-[1-(6-methylindol-3-ylmethyl) pyrrolidin-3-yl]acetamide hydrochloride

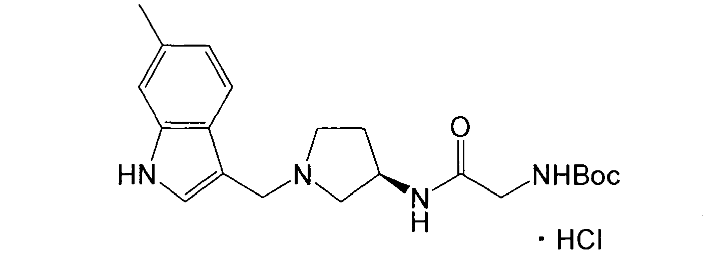

To (*R*)-2-(*t*-butoxycarbonylamino)-*N*-pyrrolidin-3-ylacetamide (36.50 g) were added 6-methylgramine (29.65 g) and 2-propanol (500 mL), and the mixture was stirred at reflux temperature to remove the solvent. When the solvent was almost distilled off, 2-propanol (500 mL) was added and further distilled off. After this procedure was repeated three times, the solvent was distilled off under reduced pressure. The residue was diluted with 2-propanol (313 mL), and here a solution prepared by mixing concentrated hydrochloric acid (13 mL) and 2-propanol (187 mL) was added dropwise with stirring, and this solution was left standing overnight. Crystals precipitated were filtered and dried to obtain the crude product, which was recrystallized by dissolving in methanol (160 mL) with heating followed by addition of 2-propanol (160 mL) to obtain the title compound (41.01 g).
¹H NMR (200 MHz, DMSO-*d*₆); δ 1.37 (s, 9H), 1.75-2.00 (m, 1H), 2.00-2.20 (m, 1H), 2.40 (s, 3H), 2.85-3.25 (m, 2H), 3.25-3.60 (m, 4H), 4.20-4.60 (m, 1H), 4.48 (s, 2H), 6.90-7.00 (m, 1H), 6.93 (d, *J =* 7.0 Hz, 1H), 7.22 (s, 1H), 7.53 (s, 1H), 7.67 (d, *J* = 7.0 Hz, 1H), 8.19-8.34 (m, 1H), 10.45-10.70 (m, 1H), 11.32 (s, 1H).

### Example 17

### Synthesis of (R)-2-amino-N-[1-(indol-3-ylmethyl)pyrrolidin-3-yl]acetamide dihydrochloride

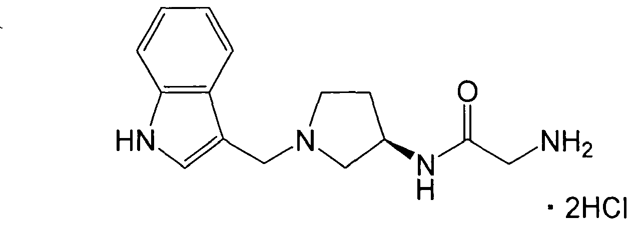

(*R*)-2-(*t*-Butoxycarbonylamino)-*N*-[1-(indol-3-ylmethyl)pyrrolidin-3-yl]acetamide hydrochloride (2.59 g) was dissolved in methanol (15 mL). Here 4 M hydrogen chloride /1,4-dioxane solution (15 mL) was added, and the mixture was stirred at room temperature for 1 hr. After distilling off the solvent under reduced pressure, the residue was washed in 1,4-dioxane (30 mL) and filtered to obtain the title compound (2.40 g) as brown solid. The purity of the title compound was determined with HPLC/MS (98%). The observed molecular weight is as follows: ESI/MS *m*/*e* 273.3 (M⁺+H, C₁₅H₂₀N₄O).

### Example 18

### Synthesis of (R)-2-{[2-amino-5-(trifluoromethoxy)phenyl]carbonylamino}-N-[1-(indol-3-ylmethy)pyrrolidin-3-yl]acetamide

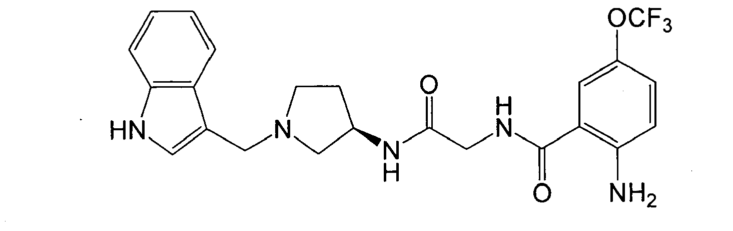

In tetrahydrofuran (50 mL) were dissolved (*R*)-2-amino-*N*-[1-(indol-3-ylmethyl)pyrrolidin-3-yl]acetamide dihydrochloride (2.27 g), 5-trifluoromethoxyanthranilic acid (1.40 g), 1-hydroxy-1,2,3-benzotriazole (0.94 g) and 1-ethyl-3-(3-dimethylaminopropyl)carbodiimide hydrochloride (1.33 g). Here triethylamine (2.73 mL) was added, and the mixture was stirred at room temperature overnight. Chilled water (50 mL) was added and the resultant insoluble material was filtered to obtain the title compound (1.94 g).
¹H NMR (400 MHz, CHCl₃-*d*); δ 1.57-1.68 (m, 1H), 2.22-2.37 (m, 2H), 2.54 (dd, *J*= 8.2 and 5.9 Hz, 1H), 2.71 (d, *J =* 8.2 Hz, 1H), 2.96-3.01 (m, 1H), 3.77 (d, *J* = 13.2 Hz, 1H), 3.87 *(d, J =* 13.2 Hz, 1H), 3.96 (d, *J =* 6.3 Hz, 2H), 4.46-4.48 (m, 1H), 5.47 (s, 2H), 6.50 (d, *J* = 6.3 Hz, 1H), 6.63 (d, *J*= 8.9 Hz, 1H), 6.85 (s, 1H), 7.08-7.29 (m, 5H), 7.37 (d, *J*= 7.8 Hz, 1H), 7.68 (d, *J*= 7.8 Hz, 1H), 8.15 (s, 1H).

### Example 19

### Synthesis of (R)-2-{[2-amino-5-(trifluoromethoxy)phenyl]carbonylamino}-N- [1-(6-methylindol -3-ylmethyl)pyrrolidin-3-yl] acetamide

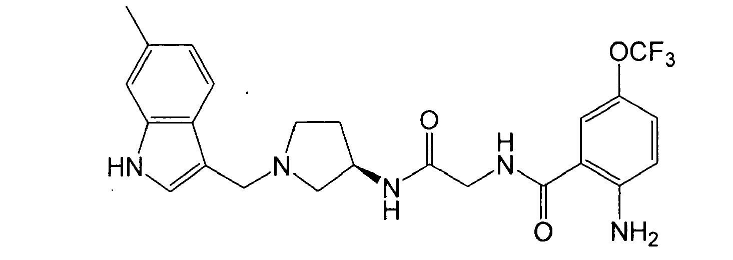

(*R*)-2-(*t*-Butoxycarbonylamino)-*N*-[1-(6-methylindol-3-ylmethyl)pyrrolidin-3-yl]acetamide hydrochloride(30.22 g) was dissolved in methanol (150 mL). Here 4 M hydrogen chloride/1,4-dioxane solution (26.8 mL) was added, and the mixture was stirred at 50°C for 1 hr. After the solvent was distilled off under reduced pressure, the residue was dissolved in tetrahydrofuran (300 mL). To this solution were added triethylamine (27.4 mL), 5-trifluoromethoxyanthranilic acid (15.80 g), 1-hydroxy-1,2,3-benzotriazole (10.94 g) and 1-ethyl-3-(3-dimethylaminopropyl)carbodiimide hydrochloride (13.70 g), and the mixture was stirred at 50°C overnight. After the solvent was distilled off, water (100 mL) and ethyl acetate (200 mL) were added. The organic layer was separated and the aqueous layer was extracted with ethyl acetate (200 mL). The combined organic layers were washed with 1 M aqueous sodium hydroxide (200 mL) and saturated brine (200 mL), dried over anhydrous magnesium sulfate, filtered to remove the drying agent, and concentrated under reduced pressure to obtain the crude product, which was further purified to obtain the title compound (17.06 g).
¹H NMR (200 MHz, DMSO-d₆); δ 1.45-1.65 (m, 1H), 1.95-2.20 (m, 1H), 2.28-2.41 (m, 2H), 2.37 (s, 3H), 2.61-2.69 (m, 2H), 3.68 (s, 2H), 3.75 (d, *J*= 5.5 Hz, 2H), 4.05-4.25 (m, 1H), 6.64 (s, 2H), 6.75 (d, *J* = 8.8 Hz, 1H), 6.80 (d, *J* = 8.1 Hz, 1H), 7.12 (s, 2H), 7.15 (d, *J* = 8.8 Hz, 1H), 7.47 (d, *J* = 8.1 Hz, 1H), 7.52 (s, 1H), 8.04 (d, *J* = 7.3 Hz, 1H), 8.51 (t, *J* = 5.5 Hz, 1H), 10.72 (s, 1H).

### Example 20

### Synthesis of (R)-2-(t-butoxycarbonylamino)-N [1-(6-methylindol-3-ylmethyl)pyrrolidin -3-yl]acetamide monohydrochloride

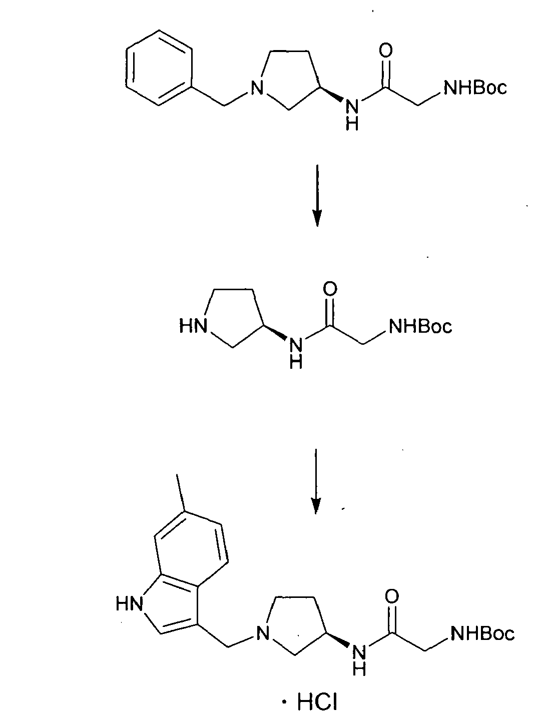

(*R*)-1-Benzyl-3-aminopyrrolidine (100 g) was dissolved in ethyl acetate (1350 mL). Here were added *N*-Boc-glycine (104.2 g), 1-hydroxy-1,2,3-benzotriazole (72.0 g), 1-ethyl-3-(3-dimethylaminopropyl)carbodiimide hydrochloride (115.2 g) and triethylamine (84.6 mL), and the mixture was stirred at room temperature for 5 hr. After reaction, water (500 mL) was added to the reaction mixture, and the organic layer was separated and washed with 5% aqueous sodium hydrogencarbonate (500 mL) twice and then 15% aqueous sodium chloride (500 mL). The organic layer containing the reaction product (*R*)-2-(*t*-butoxycarbonylamino)-*N*-(1-benzylpyrrolidin-3-yl)acetamide was used for the subsequent reaction. To this solution were added 10% Pd/C (4.8 g) and ethanol (1.0 L). The atmosphere in the reaction vessel was replaced with hydrogen and the mixture was stirred at 70°C for 2 hr. Pd/C was filtered and washed with ethanol (200 mL). The filtrate and the washings were combined and concentrated to one third of the initial volume under reduced pressure to obtain a solution containing (*R*)-2-(*t*-butoxycarbonylamino)-*N*-pyrrolidin-3-ylacetamide, which was immediately used for the subsequent reaction.

To this solution were added 6-methylgramine (106.7 g) and toluene (800 mL), and the mixture was heated under reflux for reaction for 4 hr. After the solution was cooled, methanol (200 mL) was added to it, and a solution consisting of concentrated hydrochloric acid (47 mL) and methanol (200 mL) was added dropwise at 50°C. Further, 2-propanol (500 mL) was added dropwise while refluxing. The solution was cooled to 0°C and the resultant crystals were filtered. Yield 113.2 g.
¹H NMR (200 MHz, DMSO-*d*₆); δ 1.37 (s, 9H), 1.75-2.00 (m, 1H), 2.00-2.20 (m, 1H), 2.40 (s, 3H), 2.85-3.25 (m, 2H), 3.25-3.60 (m, 4H), 4.20-4.60 (m, 1H), 4.48 (s, 2H), 6.90-7.00 (m, 1H), 6.93 (d, *J* = 7.0 Hz, 1H), 7.22 (s, 1H), 7.53 (s, 1H), 7.67 (d, *J =* 7.0 Hz, 1H), 8.19-8.34 (m, 1H), 10.45-10.70 (m, 1H), and 11.32 (s, 1H).

### Example 21

### Synthesis of (R)-2- {[2-amino-5-trifluoromethoxy)phenyl]carbonylamino}-N-[1-(6-methylindol -3-ylmethyl)pyrrolidin-3-yl]acetamide

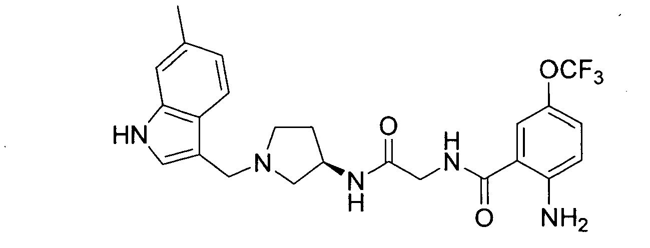

(*R*)-2-(*t*-butoxycarbonylamino)-*N* [1-(6-methylindol-3-ylmethyl)pyrrolidin-3-yl]acetamide hydrochloride(10.57 g) was dissolved in methanol (25 mL). Here 4 M hydrogen chloride/ethyl acetate solution (26.8 mL) was added and the mixture was stirred at 50°C for 2 hr. After the reaction mixture was cooled, triethylamine (37.5 mL) and ethyl acetate (75 mL) were added. Here were added 5-trifluoromethoxyanthranilic acid (4.75 g), 1-hydroxy-1,2,3-benzotriazole (3.38 g) and 1-ethyl-3-(3-dimethylaminopropyl)carbodiimide hydrochloride (4.79 g), and the mixture was kept at room temperature for reaction for 2 hr. Methanol (25 mL) and ethyl acetate (75 mL) were added to the reaction mixture, and this solution was washed with aqueous sodium hydroxide. Dilute hydrochloric acid was added to separate the solution into three layers. The top layer was discarded, and ethanol (62.5 mL) and aqueous sodium hydroxide were added to the remaining two (middle and bottom) layers to obtain the title compound (6.58 g).
¹H NMR (200 MHz, DMSO-*d*₆); δ 1.45-1.65 (m, 1H), 1.95-2.20 (m, 1H), 2.28-2.41 (m, 2H), 2.37 (s, 3H), 2.61-2.69 (m, 2H), 3.68 (s, 2H), 3.75 (d, *J=* 5.5 Hz, 2H), 4.05-4.25 (m, 1H), 6.64 (s, 2H), 6.75 (d, *J =* 8.8 Hz, 1H), 6.80 (d, *J =* 8.1 Hz, 1H), 7.12 (s, 2H), 7.15 (d, *J* = 8.8 Hz, 1H), 7.47 (d, *J* = 8.1 Hz, 1H), 7.52 (s, 1H), 8.04 (d, *J =* 7.3 Hz, 1H), 8.51 (t, *J =* 5.5 Hz, 1H), 10.72 (s, 1H).

### INDUSTRIAL APPLICABILITY

According to the present invention, the aminopyrrolidine derivatives disclosed in WO 99/25686, which are useful as chemokine receptor antagonists, can be produced easily at low cost. This production method is satisfactory as industrial synthetic process with respect to facility, operation and environmental problem.

Further, said aminopyrrolidine derivatives can be readily produced using the anthranilamide derivatives of the present invention as intermediates. The production method via these compounds is satisfactory as industrial synthetic process with respect to facility, operation and environmental problem. Further, according to the present invention, said anthranilamide derivatives themselves can be readily produced.

Namely, the anthranilamide derivatives of the present invention can be used as intermediates for producing pharmaceuticals, and aminopyrrolidine derivatives obtained by the production method of the present invention can be used as pharmaceuticals.

## Claims

1. A producing method for aminopyrrolidine derivatives or salts thereof comprising reaction steps 1 and 2 represented by the following reaction formula (I) with the proviso that reaction step 2 is unnecessary if both R¹ and R² are hydrogen: 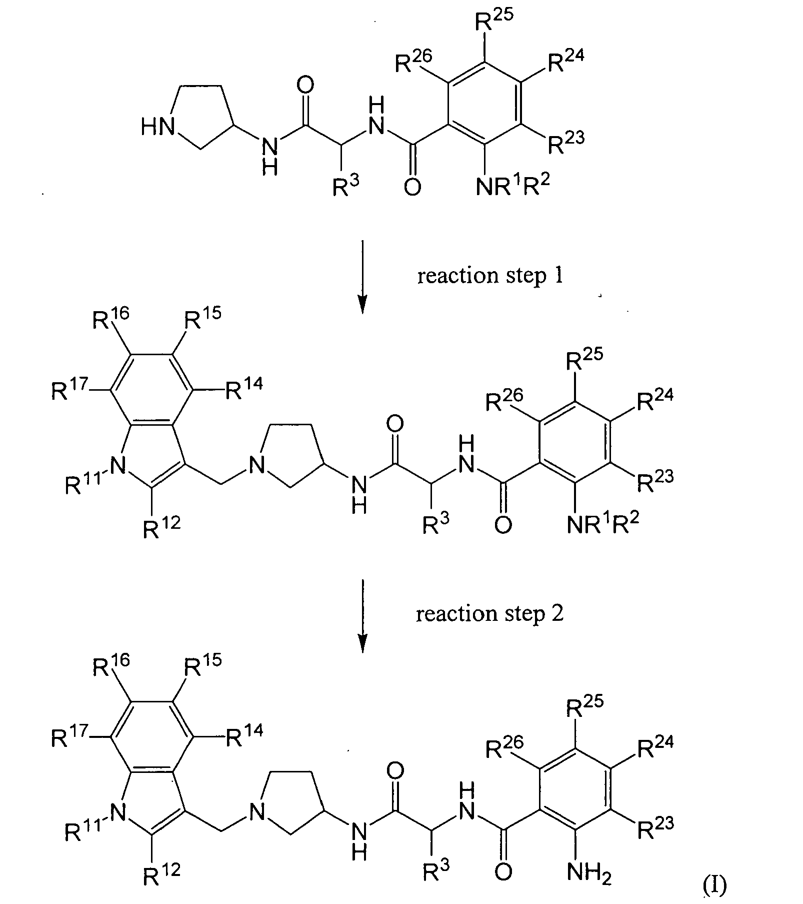 wherein R¹ and R² represent independently hydrogen or a protecting group for amino group (wherein R¹ and R² may , taken together, form a cyclic structure);
R³ represents hydrogen or C₁-C₆ alkyl;
R¹¹ represents hydrogen, C₁-C₆ alkyl or C₂-C₇ alkanoyl;
R¹², R¹⁴, R¹⁵, R¹⁶ and R¹⁷ represent independently hydrogen, halogen, optionally halogenated C₁-C₆ alkyl, optionally halogenated C₁-C₆ alkoxy, hydroxyl or C₂-C₇ alkoxycarbonyl; and R²³, R²⁴, R²⁵ and R²⁶ represent independently hydrogen, halogen, optionally halogenated C₁-C₆ alkyl, optionally halogenated C₁-C₆ alkoxy or hydroxyl.

2. The production method according to claim 1, wherein the protecting group for amino group as R¹ or R² is methoxycarbonyl, *t*-butoxycarbonyl, benzyloxycarbonyl, allyloxycarbonyl, formyl, acetyl, benzoyl, methyl, ethyl, allyl, benzenesulfonyl or phthaloyl, wherein, when said protecting group for amino group contains an aromatic ring, the aromatic ring may be optionally substituted with one or more of nitro, amino, C₁-C₆ alkyl, C₁-C₆ alkoxy or halogen.

3. The production method according to claim 1, wherein either of R¹ and R² is hydrogen and the other is *t*-butoxycarbonyl.

4. The production method according to any one of claims 1 to 3, wherein reaction step 1 is reaction of an indole derivative having no substituent at the 3-position in the presence of a synthon of formaldehyde.

5. The production method according to claim 4, wherein the synthon of formaldehyde is one or more of a compound selected from formalin, paraformaldehyde and trioxane.

6. The production method according to any one of claims 1 to 3, wherein reaction step 1 is reaction of an indole derivative having a dialkylaminomethyl group at the 3-position.

7. The production method according to any one of claims 1 to 6, wherein reaction step 2 is removal of the protection group for the amino group by acid hydrolysis.

8. The production method according to any one of claims 1 to 6, wherein reaction step 2 involves treatment with hydrogen chloride in organic solvent.

9. A method for producing aminopyrrolidine derivatives or salts thereof comprising a condensation step represented by the following reaction formula (II), wherein the condensation step is performed by treatment with an anthranilic acid derivative in an aprotic solvent in the presence of a condensing agent: 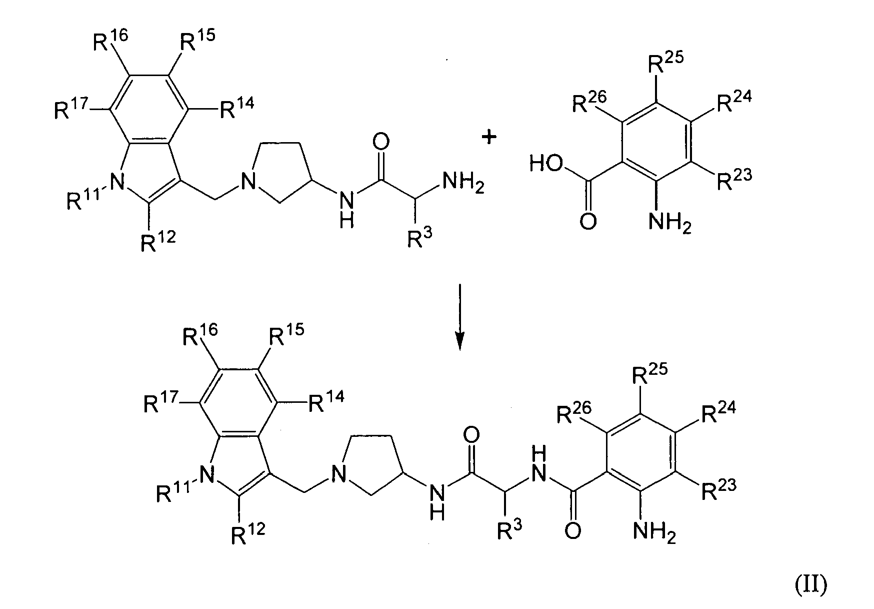 wherein R³ represents hydrogen or C₁-C₆ alkyl;
R¹¹ represents hydrogen, C₁-C₆ alkyl or C₂-C₇ alkanoyl;
R¹², R¹⁴, R¹⁵, R¹⁶ and R¹⁷ represent independently hydrogen, halogen, optionally halogenated C₁-C₆ alkyl, optionally halogenated C₁-C₆ alkoxy, hydroxyl or C₂-C₇ alkoxycarbonyl; and
R²³, R²⁴, R²⁵ and R²⁶ represent independently hydrogen, halogen, optionally halogenated C₁-C₆ alkyl, optionally halogenated C₁-C₆ alkoxy or hydroxyl.

10. The production method according to claim 9, wherein the condensing agent is one or more of a compound selected from 1,3-dicyclohexylcarbodiimide, isobutyl chloroformate, pivaloyl chloride, isovaleryl chloride, 1-ethyl-3-(3-dimethylaminopropyl)carbodiimide, 1-cyclohexyl-3-morpholinoethylcarbodiimide,
1-cyclohexyl-3-(4-diethylaminocyclohexyl)carboximide, *N*,*N'*-carbonyldiimidazole and 2-chloro-1,3-dimethylimidazolinium chloride.

11. The production method according to claim 9, wherein the condensing agent is 1-ethyl-3-(3-dimethylaminopropyl)carbodiimide hydrochloride.

12. The production method according to any one of claims 9 to 11, wherein, in said condensation step, are additionally used one or more of an additive selected from p-nitrophenol, hydroxysuccinimide, hydroxyphthalimide, 1-hydroxy-1,2,3-benzotriazole, 3-hydroxy-4-oxo-3,4-dihydro-1,2,3-benzotriazine, *N*-hydroxy-5-norbornene-2,3-dicarboximide and ethyl 2-hydroxyimino-2-cyanoacetate.

13. The production method according to any one of claims 9 to 11, wherein, in said condensation step, 1-hydroxy-1,2,3-benzotriazole is additionally used as an additive.

14. The production method according to any one of claims 9 to 13, wherein, in said condensation step, triethylamine is additionally used.

15. The production method according to any one of claims 9 to 14, which further comprises a deprotection step represented by the following reaction step 4: 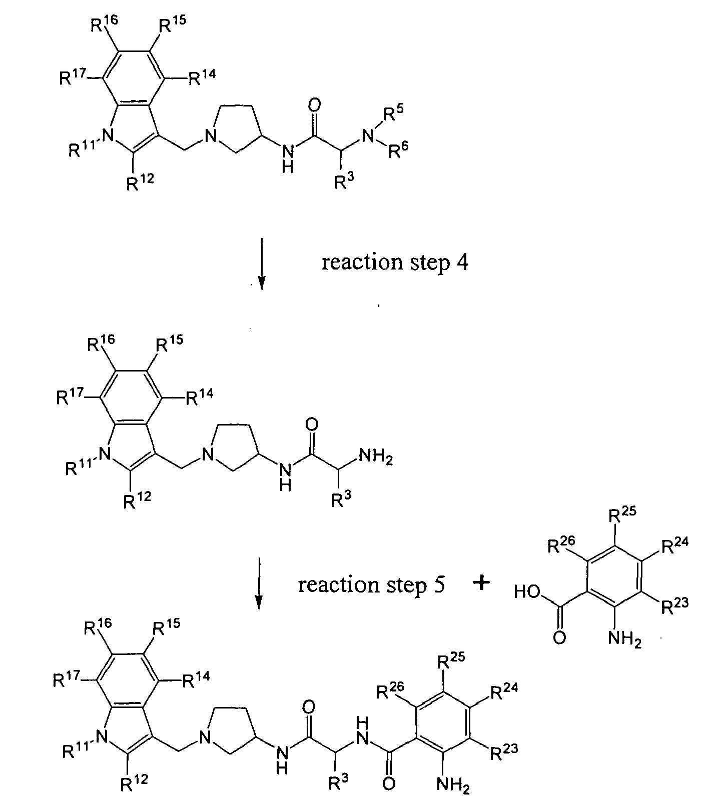 wherein R³, R¹¹, R¹², R¹⁴, R¹⁵, R¹⁶, R¹⁷, R²³, R²⁴, R²⁵ and R²⁶ are as defined in reaction formula (II);
R⁵ and R⁶ represent independently hydrogen or a protecting group for amino group (wherein R⁵ and R⁶ may, taken together, form a cyclic structure) except for the case where R⁵ and R⁶ are simultaneously hydrogen.

16. The production method according to claim 15, wherein said reaction step 4 involves treatment with hydrogen chloride in organic solvent.

17. The production method according to either claim 15 or 16, which further comprises an introduction step of an indole derivative represented by the following reaction step 3: 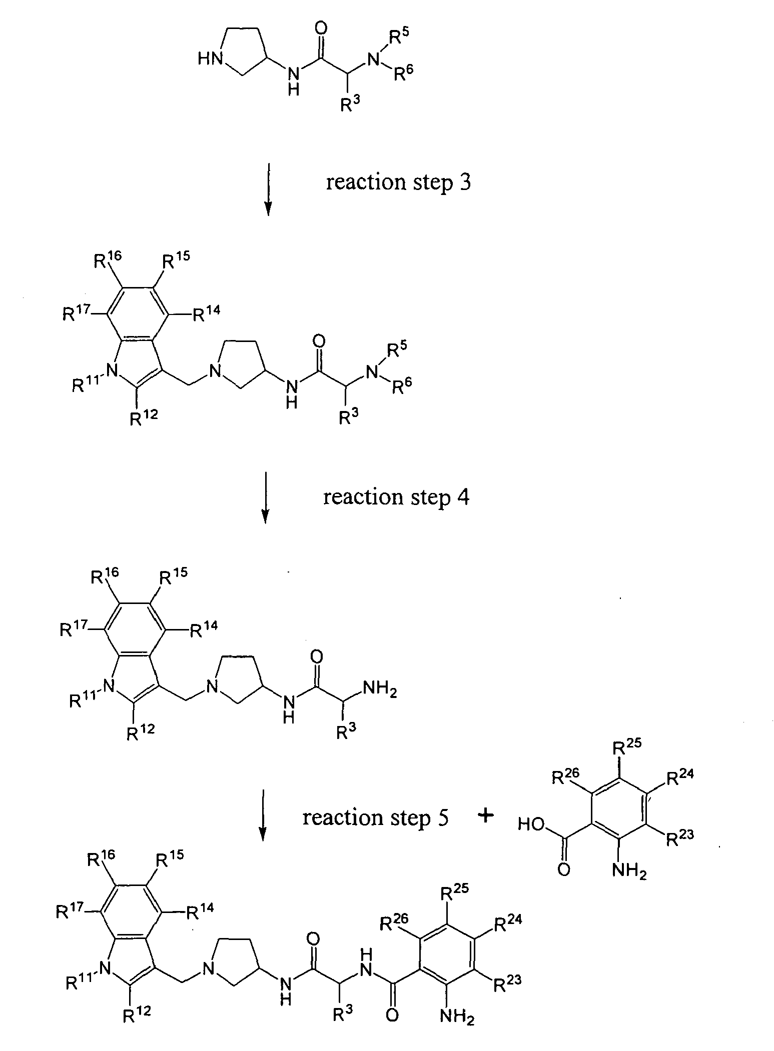 wherein R³, R⁵, R⁶, R¹¹, R¹², R¹⁴, R¹⁵, R¹⁶, R¹⁷, R²³, R²⁴, R²⁵ and R²⁶ are as defined above.

18. The production method according to claim 17, wherein said reaction step 3 is reaction of an indole derivative having no substituent at the 3-position in the presence of a synthon of formaldehyde.

19. The production method according to claim 18, wherein the synthon of formaldehyde is formalin.

20. The production method according to claim 17, wherein said reaction step 3 is reaction of an indole derivative substituted with a dialkylaminomethyl group at the 3-position.

21. The production method according to any one of claims 17 to 20, which further comprises a removal step of a benzyl group represented by the following reaction step 2: 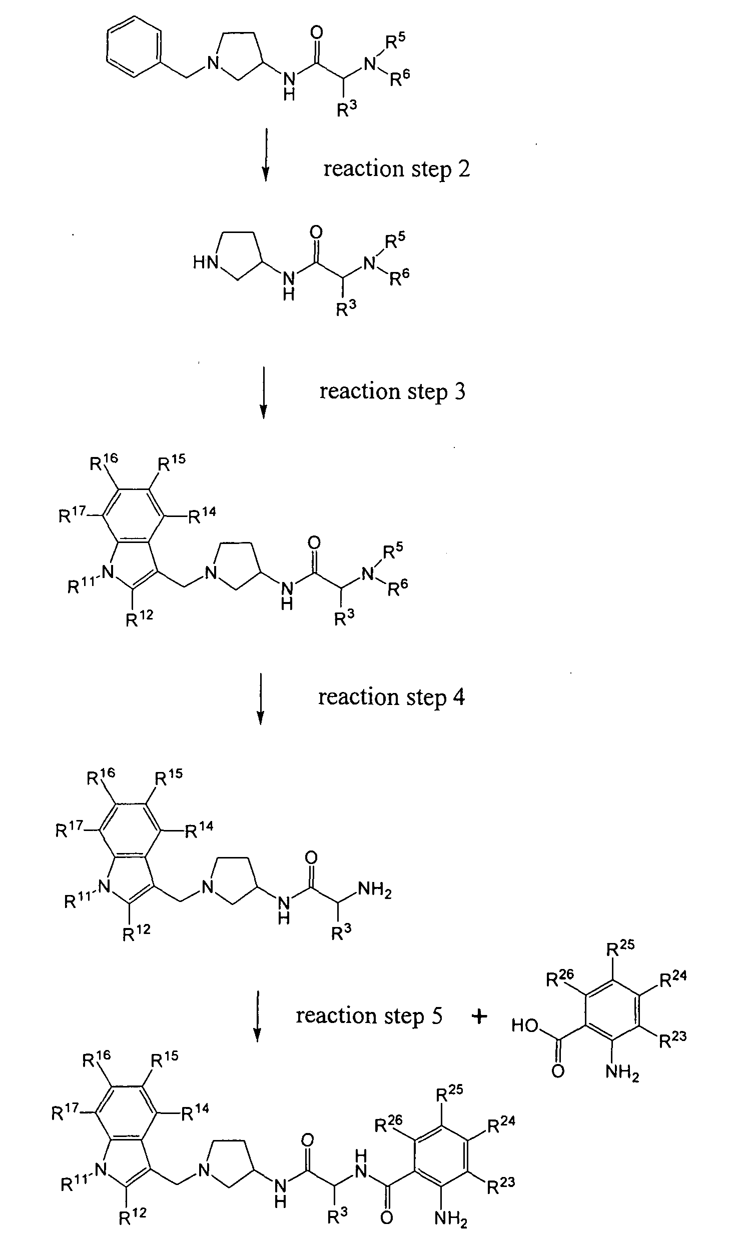 wherein R³ R⁵, R⁶, R¹¹, R¹², R¹⁴, R¹⁵, R¹⁶, R¹⁷, R²³, R²⁴, R²⁵ and R²⁶ are as defined above.

22. The production method according to claim 21, wherein, in said reaction step 2, a hydrogen source is used in the presence of palladium catalyst.

23. The production method according to claim 22, wherein the hydrogen source is gaseous hydrogen.

24. The production method according to any one of claims 21 to 23, which further comprises a condensation step with an amino acid derivative represented by the following reaction step 1: 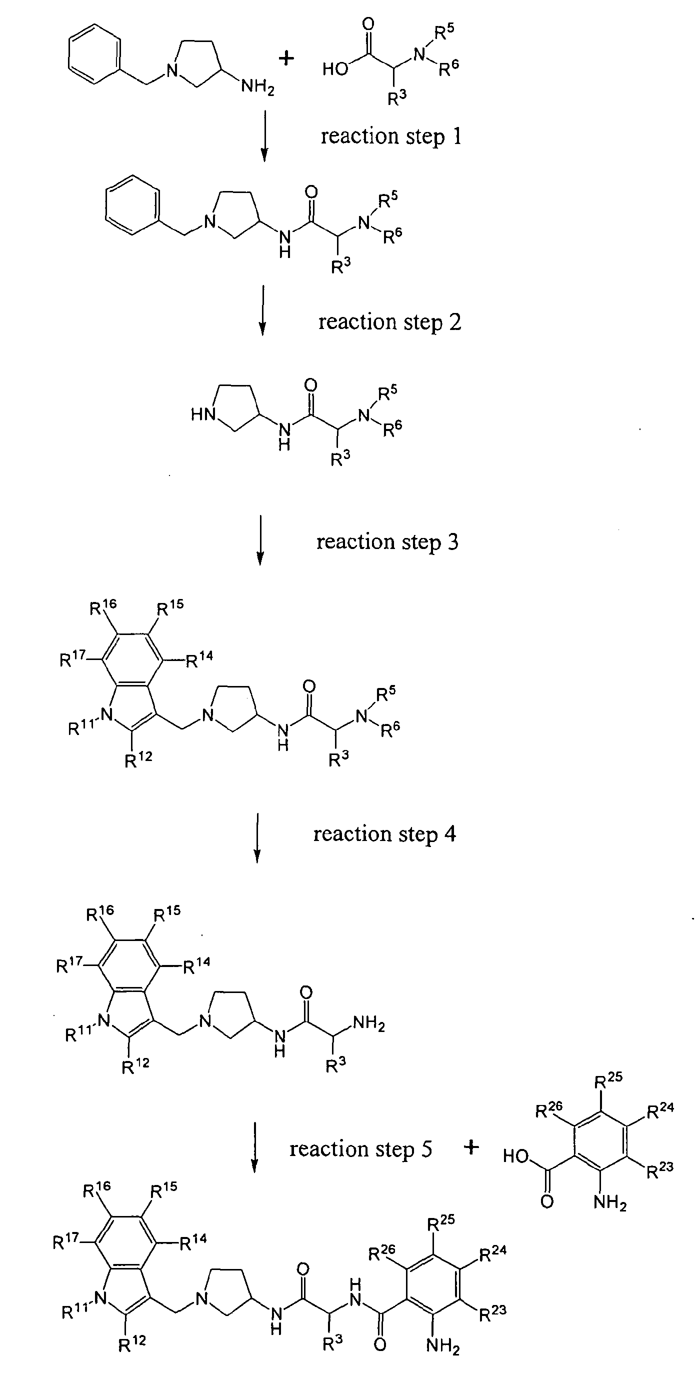 wherein R³, R⁵, R⁶, R¹¹, R¹², R¹⁴, R¹⁵, R¹⁶, R¹⁷, R²³, R²⁴, R²⁵ and R²⁶ are as defined above.

25. The production method according to claim 24, wherein, in said reaction step 1, are used one or more of a condensing agent selected from 1,3-dicyclohexylcarbodiimide, isobutyl chloroformate, pivaloyl chloride, isovaleryl chloride, 1-ethyl-3-(3-dimethylaminopropyl)carbodiimide,1-cyclohexyl-3-morpholinoethylcarbodiimide, 1-cyclohexyl-3-(4-diethylaminocyclohexyl)carboximide, *N,N'*-carbonyldiimidazole and 2-chloro-1,3-dimethylimidazolinium chloride.

26. The production method according to claim 24, wherein, in said reaction step 1, 1-ethyl-3-(3-dimethylaminopropyl)carbodiimide is used as a condensing agent.

27. The production method according to any one of claims 24 to 26, wherein, in said reaction step 1, are additionally used one or more of an additive selected from *p*-nitrophenol, hydroxysuccinimide, hydroxyphthalimide, 1-hydroxy-1,2,3-benzotriazole, 3-hydroxy-4-oxo-3,4-dihydro-1,2,3-benzotriazine, *N*-hydroxy-5-norbomene-2,3-dicarboximide and ethyl 2-hydroxyimino-2-cyanoacetate.

28. The production method according to any one of claims 24 to 26, wherein, in said reaction step 1, 1-hydroxy-1,2,3-benzotriazole is additionally used as an additive.

29. The production method according to any one of claims 24 to 28, wherein, in said reaction step 1, triethylamine is additionally used.

30. The production method according to any one of claims 15 to 29, wherein the protecting group for amino group as R⁵ and R⁶ is methoxycarbonyl, *t*-butoxycarbonyl, benzyloxycarbonyl, allyloxycarbonyl, fonnyl, acetyl, benzoyl, methyl, ethyl, allyl, benzenesulfonyl or phthaloyl, wherein, when said protecting group for the amino group contains an aromatic ring, the aromatic ring may be optionally substituted with one or more of nitro, amino, C₁-C₆ alkyl, C₁-C₆ alkoxy or halogen.

31. The production method according to any one of claims 15 to 29, wherein either of R⁵ and R⁶ is hydrogen and the other is *t*-butoxycarbonyl.

32. The production method according to any one of claims 1 to 31, wherein R³ is hydrogen.

33. The production method according to any one of claims 1 to 32, wherein R¹¹, R¹², R¹⁴, R¹⁵ and R¹⁷ are all hydrogen.

34. The production method according to any one of claims 1 to 33, wherein R¹⁶ is methyl.

35. The production method according to any one of claims 1 to 34, wherein R²³, R²⁴ and R²⁶ are all hydrogen.

36. The production method according to any one of claims 1 to 35, wherein R²⁵ is trifluoromethoxy.

37. A compound or a salt thereof represented by the following formula (III): 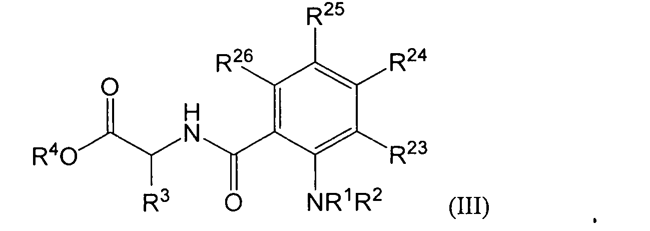 wherein R¹ and R² represent independently hydrogen or a protecting group for amino group (wherein R¹ and R² may, taken together, form a cyclic structure);
R³ represents hydrogen or C₁-C₆ alkyl;
R⁴ represents hydrogen or C₁-C₆ alkyl; and
R²³, R²⁴, R²⁵ and R²⁶ represent independently hydrogen, halogen, optionally halogenated C₁-C₆ alkyl, optionally halogenated C₁-C₆ alkoxy or hydroxyl.

38. The compound or a salt thereof according to claim 37, wherein said protecting group of amino group as R¹ and R² is methoxycarbonyl, *t*-butoxycarbonyl, benzyloxycarbonyl, allyloxycarbonyl, formyl, acetyl, benzoyl, methyl, ethyl, allyl, benzenesulfonyl or phthaloyl, wherein, when said protecting group for the amino group contains an aromatic ring, the aromatic ring may be substituted with one or more of nitro, amino, C₁-C₆ alkyl, C₁-C₆ alkoxy or halogen.

39. The compound or a salt thereof according to claim 37, wherein either of R¹ and R² is hydrogen and the other is hydrogen, *t*-butoxycarbonyl or benzyloxycarbonyl.

40. The compound or a salt thereof according to any one of claims 37 to 39, wherein R³ is hydrogen.

41. The compound or a salt thereof according to any one of claims 37 to 40, wherein R⁴ is hydrogen.

42. The compound or a salt thereof according to any one of claims 37 to 41, wherein R²³, R²⁴ and R²⁶ are all hydrogen.

43. The compound or a salt thereof according to any one of claims 37 to 42, wherein R²⁵ is C₁-C₆ alkoxy substituted with halogen.

44. The compound or a salt thereof according to any one of claims 37 to 42, wherein R²⁵ is trifluoromethoxy.

45. A production method of an anthranilamide derivative or a salt thereof comprising a reaction step represented by the following formula (IV): 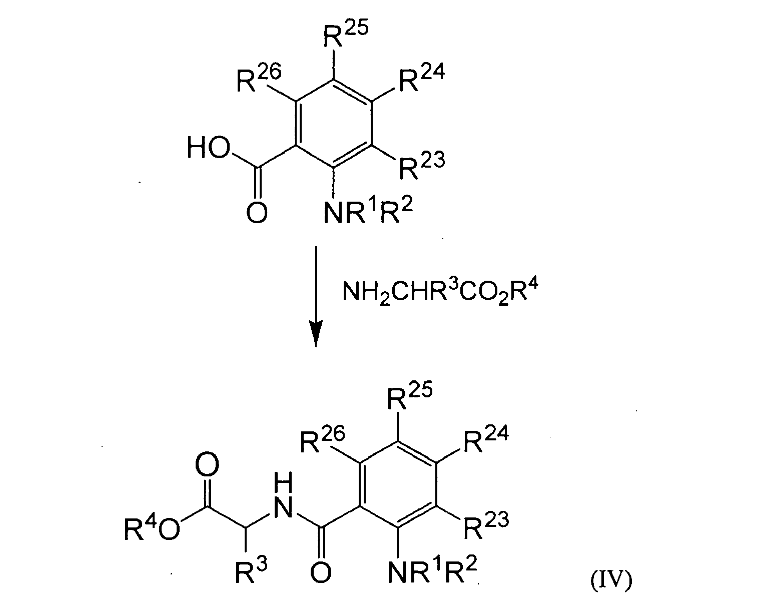 wherein:
R¹ and R² represent independently hydrogen or a protecting group for amino group (wherein R¹ and R² may, taken together, form a cyclic structure);
R³ represents hydrogen or C₁-C₆ alkyl;
R⁴ represents hydrogen or C₁-C₆ alkyl;
R²³, R²⁴, R²⁵ and R²⁶ represent independently hydrogen, halogen, optionally halogenated C₁-C₆ alkyl, optionally halogenated C₁-C₆ alkoxy or hydroxyl.

46. The production method according to claim 45 which further comprises a reaction step represented by the first step in the following reaction formula: 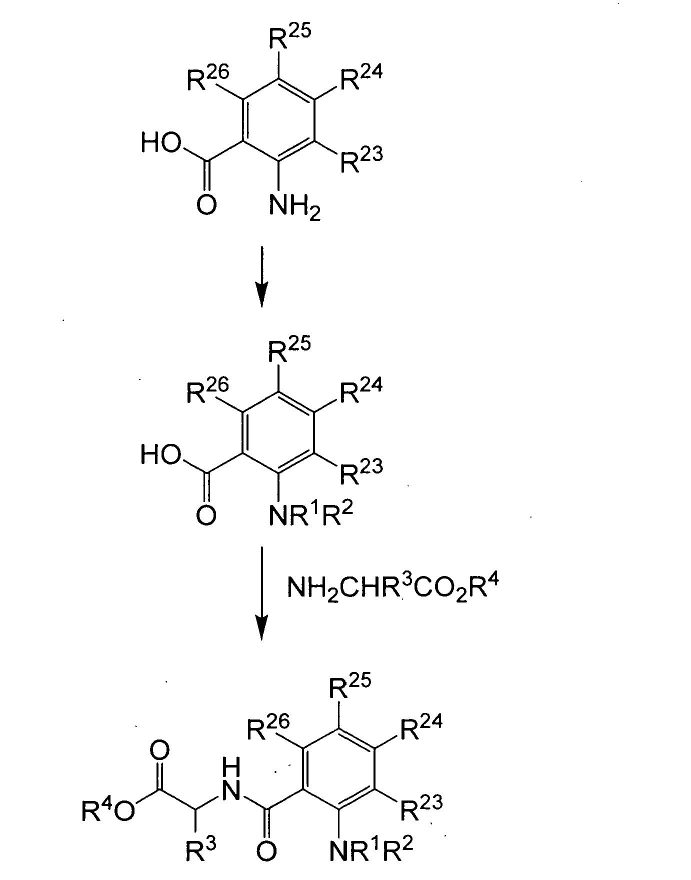 wherein R¹, R², R³, R⁴, R²³, R²⁴, R²⁵ and R²⁶ are as defined above.

47. The production method according to either claim 45 or 46, wherein the protecting group for amino group as R¹ or R² is methoxycarbonyl, *t*-butoxycarbonyl, benzyloxycarbonyl, allyloxycarbonyl, formyl, acetyl, benzoyl, methyl, ethyl, allyl, benzenesulfonyl or phthaloyl, wherein, when said protecting group for the amino group contains an aromatic ring, the aromatic ring may be substituted with one or more of nitro, amino, C₁-C₆ alkyl, C₁-C₆ alkoxy or halogen.

48. The production method according to either claim 45 or 46, wherein either of R¹ and R² is hydrogen and the other is hydrogen, *t*-butoxycarbonyl or benzyloxycarbonyl.

49. The production method according to any one of claims 45 to 48, wherein R³ is hydrogen.

50. The production method according to any one of claims 45 to 49, wherein R²³, R²⁴ and R²⁶ are all hydrogen.

51. The production method according to any one of claims 45 to 50, wherein R²⁵ is C₁-C₆ alkoxy substituted with halogen.

52. The production method according to any one of claims 45 to 50, wherein R²⁵ is trifluoromethoxy.
